(19) Europäisches Patentamt
European Patent Office
Office européen des brevets

(11) Veröffentlichungsnummer: **0 073 502**
**B1**

(12) # EUROPÄISCHE PATENTSCHRIFT

(45) Veröffentlichungstag der Patentschrift: **14.05.86**

(51) Int. Cl.⁴: **C 07 F 7/00**, C 07 F 7/28, A 61 K 31/28, A 61 K 31/74, A 61 K 9/18

(21) Anmeldenummer: **82107914.2**

(22) Anmeldetag: **28.08.82**

(54) **Antineoplastisch wirkende Metallkomplexe und diese enthaltende Arzneimittel.**

(30) Priorität: **02.09.81 DE 3134709**

(43) Veröffentlichungstag der Anmeldung:
**09.03.83 Patentblatt 83/10**

(45) Bekanntmachung des Hinweises auf die Patenterteilung:
**14.05.86 Patentblatt 86/20**

(84) Benannte Vertragsstaaten:
**AT BE CH DE FR GB IT LI LU NL SE**

(56) Entgegenhaltungen:
**FR - A - 2 079 013**
**FR - A - 2 121 289**
**GB - A - 1 473 335**
**US - A - 3 334 067**

**CHEMICAL ABSTRACTS, Band 58, Nr. 4, Februari 18, 1963, Ref. 3343h Columbus, Ohio, US D.M. PURI et al.: "Derivatives of titanium with compds. having bidentate ligands. VI. Derivs. with benzoylacetone"**
**JOURN. CHEM. SOC. (A) 1970 Siete 904-7 U.B. SAXENA et al.: "Reactions of Zirconium Isopropoxide with beta-Di-ketones and beta-Keto-esters"**
**JOURNAL OF AMER. CHEM. SOC. Band 79, Seiten 4344-48, 1957 AKIO YAMAMOTO et al.: "Structures of the Reaction Products of Tetraalkoxytitanium with Acetylacetone and Ethyl Acetoacetate"**

(73) Patentinhaber: **Byk Gulden Lomberg Chemische Fabrik GmbH, Byk-Gulden-Strasse 2, D-7750 Konstanz (DE)**

(72) Erfinder: **Keller, Heimo J., Angelweg 28, D-6900 Heidelberg (DE)**
Erfinder: **Keppler, Bernhard, Wolfgangstrasse 11, D-6900 Heidelberg-Kirchheim (DE)**
Erfinder: **Krüger, Uwe, Neuhauser Strasse 11, D-7750 Konstanz (DE)**
Erfinder: **Linder, Rudolf, Hermann-von-Vicaristrasse 41, D-7750 Konstanz (DE)**

**EP 0 073 502 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung bezieht sich auf antineoplastisch wirkende Metallkomplexe und diese enthaltende Arzneimittel.

Stand der Technik

Kürzlich wurde ein die Komplexverbindung cis-Diammindichloroplatin (II) enthaltendes Arzneimittel als Chemotherapeutikum gegen Krebs in den Handel gebracht. Diese unter dem International Nonproprietary Name (INN) Cisplatin bekannte Verbindung hat sich als äusserst potentes Antitumormittel, insbesondere bei der Behandlung von Hodentumoren, aber auch z.B. von Eierstocktumoren und kleinzelligen Bronchialkarzinomen erwiesen. Nachteilig an Cisplatin ist seine relativ grosse Toxizität. Besonders gravierend sind seine Nephrotoxizität sowie seine zu bleibenden Gehörschäden führende Wirkung. Nieren- und Gehörschäden werden bereits nach Verabreichung einer einzigen therapeutischen Dosis mit erheblicher Häufigkeit festgestellt. Neben der nephro- und haematotoxischen Wirkung sind für die Patienten vor allem noch die langanhaltende starke Übelkeit und der damit verbundene Brechreiz äusserst unangenehm.

Es wurden in letzter Zeit zahlreiche andere Platinkomplexe (DE-OS 2 445 418, DE-OS 2 837 237, DE-OS 2 626 559, DE-OS 2 539 179) und Komplexverbindungen anderer Übergangsmetalle als cytostatisch wirkende Mittel vorgeschlagen. In der DE-OS 2 801 335 werden einem braunen amorphen Komplex, gewonnen durch Reaktion von Ascorbinsäure mit einer Titan(III)- und einer Kupfer(II)verbindung (Molverhältnis 36:1:6) kurative und prophylaktische Wirkungen gegen u.a. Leukämie zugeschrieben. Es wurde berichtet, dass Titanocen-, Zirconocen- und Hafnocendichlorid hemmend gegenüber dem Ehrlich-Ascites-Tumor der Maus wirken [P. Köpf-Maier, B. Hesse, H. Köpf, J. Cancer Res. Clin. Oncol. 96, 43 (1980)]. In der EP-OS 49 486 werden Dihalogenobis-(1,3-diketonato)-zinn-,-titan-,-zirkon- und -hafniumverbindungen mit antineoplastischer Wirksamkeit beschrieben. In der US-A-3 334 067 wird ein Herstellungsverfahren für bei Raumtemperatur aushärtende Siloxangummis beschrieben. Als Katalysatoren werden unter anderem Bis(β-dicarbonyl)titandi($C_1$–$C_{18}$)alkoxide vorgeschlagen.

Darstellung der Erfindung

Überraschend wurde nun gefunden, dass die Metallkomplexe der allgemeinen Formel I,

$$[R^1(CH_2)_mC(O)CR^3C(O)R^2]_2M(OR^4)_{2-n}X_n \qquad (I),$$

worin

M Titan, Zirkon oder Hafnium,

$R^1$ Wasserstoff, $C_1$–$C_8$-Alkyl oder Phenyl, das durch Fluor, Chlor, Brom, Nitro, $C_1$–$C_4$-Alkyl, $C_1$–$C_4$-Alkoxy oder Trifluormethyl einfach oder mehrfach substituiert sein kann,

$R^2$ $C_1$–$C_8$-Alkyl oder Phenyl, das durch Fluor, Chlor, Brom, Nitro, $C_1$–$C_4$-Alkyl, $C_1$–$C_4$-Alkoxy oder Trifluormethyl einfach oder mehrfach substituiert sein kann,

$R^3$ Wasserstoff oder Phenyl,

$R^4$ $C_1$–$C_{18}$-Alkyl, das durch Hydroxy, $C_1$–$C_3$-Alkylamin oder Alkalisulfonatogruppen substituiert sein kann, oder $C_5$–$C_8$-Cycloalkyl, das durch $C_1$–$C_5$-Alkylgruppen, Hydroxy oder Alkalisulfonatogruppen substituiert sein kann,

X Fluor, Chlor oder Brom,

m die Zahl 0 oder 1, aber nicht 0, wenn $R^1$ die Bedeutung Wasserstoff hat, und

n die Zahl 0 oder 1,

bedeuten, und, soweit ein Rest $R^4$ eine Aminogruppe enthält, deren Hydrohalogenide eine interessante cytostatische Wirksamkeit bei günstiger therapeutischer Breite aufweisen und sich als Chemotherapeutikum zur Behandlung von Krebskrankheiten eignen.

Gegenstand der Erfindung sind daher Arzneimittel enthaltend einen oder mehrere der Metallkomplexe der vorstehenden allgemeinen Formel I, worin M, $R^1$, $R^2$, $R^3$, $R^4$, X, m und n die oben angegebenen Bedeutungen haben, insbesondere zur Bekämpfung von Krebskrankheiten.

Besonders bevorzugt sind Arzneimittel enthaltend einen oder mehrere Metallkomplexe der allgemeinen Formel I′,

$$[R^{1'}(CH_2)_{m'}C(O)CR^{3'}C(O)R^{2'}]_2M'(OR^{4'})_{2-n}X'_n \qquad (I'),$$

worin

M′ Titan, Zirkon oder Hafnium,

$R^{1'}$ Wasserstoff, $C_1$–$C_6$-Alkyl oder Phenyl, das durch Fluor, Chlor, Brom, Nitro, $C_1$–$C_2$-Alkyl, $C_1$–$C_2$-Alkoxy oder Trifluormethyl einfach oder mehrfach substituiert sein kann,

$R^{2'}$ $C_1$–$C_6$-Alkyl oder Phenyl, das durch $C_1$–$C_2$-Alkoxy substituiert sein kann,

$R^{3'}$ Wasserstoff oder Phenyl,

$R^{4'}$ $C_2$–$C_{16}$-Alkyl, das durch Hydroxy, $C_1$–$C_3$-Alkylamin oder Alkalisulfonatogruppen substituiert sein kann, oder Cyclohexyl, das durch $C_1$–$C_4$-Alkylgruppen substituiert sein kann,

X′ Fluor oder Chlor,

m′ die Zahl 0 oder 1, aber nicht 0, wenn $R^{1'}$ die Bedeutung Wasserstoff hat, und

n die Zahl 0 oder 1,

bedeuten, und, soweit ein Rest $R^{4'}$ eine Aminogruppe enthält, deren Hydrohalogenide.

Besonders bevorzugt sind Arzneimittel enthaltend einen oder mehrere Metallkomplexe der allgemeinen Formel I″,

$$[R^{1''}(CH_2)_{m''}C(O)CR^{3''}C(O)R^{2''}]_2M''(OR^{4''})_{2-n}X''_n \qquad (I''),$$

worin

M″ Titan, Zirkon oder Hafnium,

$R^{1''}$ Wasserstoff, $C_1$–$C_4$-Alkyl oder Phenyl, das durch $C_1$–$C_2$-Alkoxy substituiert sein kann,

$R^{2''}$ $C_1$–$C_4$-Alkyl oder Phenyl,

$R^{3''}$ Wasserstoff oder Phenyl,

$R^{4''}$ $C_2$–$C_{13}$-Alkyl, das durch ein Hydroxy, ein Di-$C_1$–

$C_3$-Alkylamin oder eine Alkalisulfonatogruppe substituiert sein kann, oder 1-Isopropyl-4-methyl-cyclohexyl-2,
X″ Chlor,
m″ die Zahl 1, falls $R^{1''}$ Wasserstoff bedeutet, und die Zahl 0, falls $R^{1''}$ Phenyl bedeutet,
n die Zahl 0 oder 1, bedeuten, und, soweit ein Rest $R^{4''}$ einen Aminorest enthält, deren Hydrochloride.

Besondere Ausgestaltungen der Erfindung sind Arzneimittel enthaltend Verbindungen der vorstehenden Formeln I, I′ bzw. I″, worin die Variablen $R^1$, $R^{1'}$ bzw. $R^{1''}$, $R^2$, $R^{2'}$ bzw. $R^{2''}$, $R^3$, $R^{3'}$ bzw. $R^{3''}$, $R^4$, $R^{4'}$ bzw. $R^{4''}$, X, X′ bzw. X″, m, m′ bzw. m″ und n die vorstehend angegebenen Bedeutungen haben, M, M′ bzw. M″ jedoch jeweils nur Titan bedeuten. Besonders bevorzugt sind Arzneimittel enthaltend Diethoxybis-(1-phenyl-1,3-butandionato)-titan(IV), Di-(iso-propoxy)-bis-(1-phenyl-1,3-butandionato)-titan(IV), Bis-(2-hydroxypropoxy)-bis-(1-phenyl-1,3-butandionato)-titan(IV) und/oder Diethoxybis-(3-phenyl-2,4-pentandionato)-titan(IV).

Ein weiterer Gegenstand der Erfindung ist die Verwendung der Verbindungen der allgemeinen Formeln I, I′ bzw. I″ zur Herstellung von Arzneimitteln, insbesondere von solchen zur Behandlung von Krebskrankheiten.

Die Verbindungen der allgemeinen Formel $I^a$,

$$[R^{1a}(CH_2)_{m^a}C(O)CR^{3a}C(O)R^{2a}]_2M^a(OR^{4a})_{2-n^a}X^a_{n^a} \quad (I^a),$$

worin
$M^a$ Titan, Zirkon oder Hafnium,
$R^{1a}$ Wasserstoff, $C_1$–$C_8$-Alkyl oder Phenyl, das durch Fluor, Chlor, Brom, Nitro, $C_1$–$C_4$-Alkyl, $C_1$–$C_4$-Alkoxy oder Trifluormethyl einfach oder mehrfach substituiert sein kann,
$R^{2a}$ $C_1$–$C_8$-Alkyl oder Phenyl, das durch Fluor, Chlor, Brom, Nitro, $C_1$–$C_4$-Alkyl, $C_1$–$C_4$-Alkoxy oder Trifluormethyl einfach oder mehrfach substituiert sein kann,
$R^{3a}$ Wasserstoff oder Phenyl,
$R^{4a}$ $C_1$–$C_{18}$-Alkyl, das durch Hydroxy, $C_1$–$C_3$-Alkylamin oder Alkalisulfonatogruppen substituiert sein kann, oder $C_5$–$C_8$-Cycloalkyl, das durch $C_1$–$C_5$-Alkylgruppen, Hydroxy oder Alkalisulfonatgruppen substituiert sein kann, aber nicht unsubstituiertes $C_1$–$C_5$- oder $C_{18}$-Alkyl, falls $M^a$ Titan, $R^{3a}$ Wasserstoff, $m^a$ und $n^a$ die Zahl 0 und $R^{1a}$ $C_1$- oder $C_4$-Alkyl oder Phenyl und $R^{2a}$ $C_1$–$C_6$-Alkyl oder Phenyl bedeuten, und nicht unsubstituiertes $C_3$-Alkyl, falls $M^a$ Zirkon, $R^{3a}$ Wasserstoff, $m^a$ und $n^a$ die Zahl 0 und $R^{1a}$ und $R^{2a}$ unabhängig voneinander Methyl oder Phenyl bedeuten,
$X^a$ Fluor, Chlor oder Brom,
$m^a$ die Zahl 0 oder 1, aber nicht 0, wenn $R^{1a}$ die Bedeutung Wasserstoff hat, und
$n^a$ die Zahl 0 oder 1,
bedeuten, und, soweit $R^{4a}$ eine Aminogruppe enthält, deren Hydrohalogenide sind neu und daher ein weiterer Gegenstand der Erfindung.

Bevorzugt sind Verbindungen der allgemeinen Formel $I^{a'}$,

$$[R^{1a'}(CH_2)_{m^{a'}}C(O)CR^{3a'}C(O)R^{2a'}]_2M^{a'}(OR^{4a'})_{2-n^{a'}}X^{a'}_{n^{a'}} \quad (I^{a'}),$$

worin
$M^{a'}$ Titan, Zirkon oder Hafnium,
$R^{1a'}$ Wasserstoff, $C_1$–$C_4$-Alkyl oder Phenyl, das durch $C_1$–$C_2$-Alkoxy substituiert sein kann,
$R^{2a'}$ Methyl oder Phenyl,
$R^{3a'}$ Wasserstoff oder Phenyl,
$R^{4a'}$ $C_2$–$C_{16}$-Alkyl, das durch Hydroxy, Di-$C_1$–$C_3$-Alkylamin oder eine Alkalisulfonatogruppe substituiert sein kann, oder 1-Isopropyl-4-methyl-cyclohexyl-2, aber nicht unsubstituiertes $C_2$–$C_5$-Alkyl, falls $M^{a'}$ Titan, $R^{3a'}$ Wasserstoff, $m^{a'}$ und $n^{a'}$ die Zahl 0 und $R^{1a'}$ $C_1$- oder $C_4$-Alkyl oder Phenyl bedeuten, und nicht unsubstituiertes $C_3$-Alkyl, falls $M^{a'}$ Zirkon, $R^{3a'}$ Wasserstoff, $m^{a'}$ und $n^{a'}$ die Zahl 0 und $R^{1a'}$ Methyl oder Phenyl bedeuten,
$X^{a'}$ Chlor,
$m^{a'}$ die Zahl 0 oder 1, aber nicht 0, wenn $R^{1a'}$ die Bedeutung Wasserstoff hat, und
$n^{a'}$ die Zahl 0 oder 1,
bedeuten, und, soweit $R^{4a'}$ eine Aminogruppe enthält, deren Hydrochloride.

Besonders bevorzugt sind
Chloro-(2,3-dimethyl-2,3-butandiolato)-bis-(1-phenyl-1,3-butandionato)-titan(IV),
Chloro-(2-hydroxypropanolato)-bis-(1-phenyl-1,3-butandionato)-titan(IV),
Bis-(tridecanolato)-bis-(1-phenyl-1,3-butandionato)-titan(IV) und
Diethoxy-bis-(3-phenyl-2,4-pendandionato)-titan(IV).

Unter Hydrohalogeniden werden Hydrochloride, Hydrobromide und Hydroiodide verstanden, wobei Hydrochloride bevorzugt sind.

Unter einer Alkalisulfonatogruppe wird ein A-$SO_3$-Rest, wobei A ein Alkalimetall bedeutet, verstanden. Der Natriumsulfonatorest ist bevorzugt.

Unter $C_1$–$C_{18}$-Alkylresten sind verzweigte und geradkettige Alkylreste mit eins bis achtzehn Kohlenstoffatomen zu verstehen. Alkylreste mit mehr als zehn Kohlenstoffatomen sind vorzugsweise unverzweigt.

Unter $C_1$–$C_3$-Alkylamin wird Mono- und Di-$C_1$–$C_3$-Alkylamin verstanden, wobei Diethylamin bevorzugt ist.

Unter $C_5$–$C_8$-Cycloalkyl sind Cycloalkyle mit fünf bis acht Ringkohlenstoffatomen zu verstehen, wobei Cyclohexyl bevorzugt ist.

Die Herstellung der Metallkomplexe erfolgt entweder durch Umsetzung des entsprechenden Metalltetraalkoholats mit dem entsprechenden Diketon im Molverhältnis 1:2 [A. Yamamoto, S. Kambara, J. Am. Chem. Soc. 79, 4344 (1957)] oder durch Umsetzung des entsprechenden Dihalogenobis-(diketonato)-metalls(IV) mit dem entsprechenden Alkohol oder auch dessen Alkoholat, vorzugsweise Alkalialkoholat, im Molverhältnis 1:1, wenn ein Monohalogenometall(IV)komplex (n in der allgemeinen Formel I hat die Bedeutung 1) hergestellt werden soll, oder im Molverhältnis 1:2, wenn ein Dialkoholatobisdike-

tonatometall(IV)komplex (n in der allgemeinen Formel I hat die Bedeutung 0) hergestellt werden soll [D.M. Puri, R.C. Mehrotra, J. Ind. Chem. Soc. 39, 499 (1962)]. Bei der Umsetzung eines Dihalogenobis-(diketonato)-metall(IV)komplexes mit einem Alkohol ist es zweckmässig, in Gegenwart einer Base zu arbeiten. Als Base wird vorteilhaft Ammoniak eingesetzt, das durch das Reaktionsgemisch geleitet wird. Das entstehende Ammoniumhalogenid ist in den verwendeten Lösungsmitteln nur wenig löslich und kann durch Filtrieren und/oder Zentrifugieren abgetrennt werden. Reste des Ammoniumhalogenids können aus dem Endprodukt durch Sublimation am Hochvakuum entfernt werden. Die Alkoholato-Gruppen der Alkoholato-diketonatometall(IV)komplexe können gegen andere Alkoholato-Gruppen ausgetauscht werden [U.B. Saxena et al., J. Chem. Soc. A 1970, 904; D.M. Puri, R.C. Mehrotra, J. Indian Chem. Soc. 39, 499 (1962)]. Man erhitzt den Alkoholato-diketonatometall(IV)komplex, dessen Alkohlato-Gruppen ausgetauscht werden sollen, als solchen oder in einem geeigneten Lösungsmittel zusammen mit einem Alkohol, der vorzugsweise im Überschuss eingesetzt wird. Diese Methode eignet sich vor allem zum Austausch eines niedrigen Alkoholatanions gegen das Alkoholatanion eines höheren Alkohols. In vielen Fällen kann der Austausch begünstigt werden, indem der freigesetzte Alkohol als Azeotrop abdestilliert wird. Die Reaktionen werden zweckmässigerweise in einer trockenen Schutzgasatmosphäre, vorzugsweise Stickstoffatmosphäre, und unter Verwendung von wasserfreien Ausgangsstoffen sowie gegebenenfalls sorgfältig getrockneten Lösungsmitteln durchgeführt.

Die Umsetzungen werden entweder ohne Lösungsmittel oder in inerten Lösungsmitteln, wie beispielsweise Benzol, n-Hexan, Diethylether, Methylenchlorid oder Chloroform, durchgeführt. Ohne Lösungsmittel kann vor allem dann gearbeitet werden, wenn mindestens einer der Reaktionspartner als Flüssigkeit vorliegt. Soweit einer der Reaktionspartner in dem verwendeten Lösungsmittel unlöslich ist, wird er als Suspension in diesem Lösungsmittel vorgelegt und der lösliche Reaktionspartner gelöst zugetropft.

Je nach Heftigkeit der Umsetzung wird bei Zimmertemperatur, unter Kühlung oder Erhitzen, beispielsweise am Rückfluss, gearbeitet. Zur Vervollständigung der Umsetzung kann es notwendig sein, das Reaktionsgemisch ein bis drei Tage am Rückfluss zu erhitzen. Bei Umsetzungen, bei denen Halogenwasserstoff freigesetzt wird, ist es vorteilhaft, diesen durch Durchleiten von trockenem Stickstoff durch das Reaktionsgemisch zu vertreiben. Die Reaktionsprodukte werden aus der Reaktionslösung entweder durch Einengen und/oder Abkühlen und/oder durch Zugabe fällender Lösungsmittel, insbesondere Hexan und Petrolether, ausgefällt.

Die 1,3-Diketone sind bekannt oder können nach an sich bekannten Methoden hergestellt werden. Beispielsweise können sie durch Esterkondensation des entsprechenden Arylmethylketons mit Essigsäureethylester bzw. des entsprechenden Arylessigsäureethylesters mit Natriumamid als Kondensationsmittel [J.T. Adams, C.R. Hauser, J. Amer. Chem. Soc. 66, 1220 (1944)] erhalten werden. Weiter ist es möglich, durch Zugabe des entsprechenden Arylmethylketons, gelöst in Essigsäureethylester, zu einer Suspension von Natrium in Benzol oder Toluol zu den 1,3-Diketonen zu gelangen [D.W. Brown, S.F. Dyke, M. Sainsbury, G. Hardy, J. Chem. Soc. (c) 1971, 3219]. Eine weitere Möglichkeit besteht in der Umsetzung von Arylmethylketonen mit Acetanhydrid in Gegenwart von Bortrifluorid [H.G. Walker, C.R. Hauser, J. Amer. Chem. Soc. 68, 2742 (1946)]. 1-Benzyl-1,3-diketone werden durch Kondensation des entsprechenden Phenylessigsäureethylesters mit dem entsprechenden Methylketon in Gegenwart von Natriumamid hergestellt [A. Becker, Helv. Chim. Acta 149, 1114 (1949)].

Gegenstand der Erfindung ist weiterhin ein Verfahren zur Herstellung der Verbindungen der allgemeinen Formeln $I^a$ bzw. $I^{a'}$, worin $M^a$ bzw. $M^{a'}$, $R^{1a}$ bzw. $R^{1a'}$, $R^{2a}$ bzw. $R^{2a'}$, $R^{3a}$ bzw. $R^{3a'}$, $R^{4a}$ bzw. $R^{4a'}$, $X^a$ bzw. $X^{a'}$, $m^a$ bzw. $m^{a'}$ und $n^a$ bzw. $n^{a'}$ die vorstehend angegebenen Bedeutungen haben, das dadurch gekennzeichnet ist, dass man

a) eine Tetraalkoxymetall(IV)verbindung $M^a(OR^{4a})_4$ bzw. $M^{a'}(OR^{4a'})_4$, wobei $M^a$ bzw. $M^{a'}$, $R^{4a}$ bzw. $R^{4a'}$ die vorstehend angegebenen Bedeutungen haben, unter Feuchtigkeitsausschluss in einem inerten Lösungsmittel mit einem Diketon $R^{1a}(CH_2)_{m^a}C(O)CHR^{3a}C(O)R^{2a}$ bzw. $R^{1a'}(CH_2)_{m^{a'}}C(O)CHR^{3a'}C(O)R^{2a'}$, wobei $R^{1a}$ bzw. $R^{1a'}$, $R^{2a}$ bzw. $R^{2a'}$, $R^{3a}$ bzw. $R^{3a'}$ und $m^a$ bzw. $m^{a'}$ die vorstehend angegebenen Bedeutungen haben,

b) eine Dihalogenobis-(diketonato)-metall(IV)-verbindung $[R^{1a}(CH_2)_{m^a}C(O)CR^{3a}C(O)R^{2a}]_2M^aX^a{}_2$ bzw. $[R^{1a'}(CH_2)_{m^{a'}}C(O)CR^{3a'}C(O)R^{2a'}]_2M^{a'}X^{a'}{}_2$, worin $M^a$ bzw. $M^{a'}$, $R^{1a}$ bzw. $R^{1a'}$, $R^{2a}$ bzw. $R^{2a'}$, $R^{3a}$ bzw. $R^{3a'}$, $X^a$ bzw. $X^{a'}$ und $m^a$ bzw. $m^{a'}$ die vorstehend angegebenen Bedeutungen haben, mit einem Alkohol $HOR^{4a}$ bzw. $HOR^{4a'}$ oder dem entsprechenden Alkalialkoholat, worin $R^{4a}$ bzw. $R^{4a'}$ die oben angegebenen Bedeutungen haben, umsetzt, oder

c) eine Verbindung der allgemeinen Formel $I^a$ bzw. $I^{a'}$, worin $n^a$ bzw. $n^{a'}$ 0 bedeuten und $M^a$ bzw. $M^{a'}$, $R^{1a}$ bzw. $R^{1a'}$, $R^{2a}$ bzw. $R^{2a'}$, $R^{3a}$ bzw. $R^{3a'}$, $R^{4a}$ bzw. $R^{4a'}$ und $m^a$ bzw. $m^{a'}$ die vorstehend angegebenen Bedeutungen haben, mit einem Alkohol $HOR^{4a}$ bzw. $HOR^{4a'}$, worin $R^{4a}$ bzw. $R^{4a'}$ die oben angegebenen Bedeutungen hat, umsetzt.

Die erfindungsgemässen Arzneimittel werden vor allem intravenös, aber auch intramuskulär, intraperitoneal, subkutan, rektal oder peroral verabreicht. Auch eine äusserliche Applikation ist möglich. Bevorzugt ist die Verabreichung durch intravenöse Injektion oder intravenöse Infusion.

Die Arzneimittel werden nach an sich bekannten Verfahren hergestellt, wobei die Komplexverbindungen als solche oder gegebenenfalls in Kombination mit geeigneten pharmazeutischen Trägerstoffen eingesetzt werden. Enthalten die neuen pharmazeutischen Zubereitungen neben dem Wirkstoff pharmazeutische Trägerstoffe, beträgt der Wirkstoffgehalt dieser Mischungen 0,1 bis 99,5, vorzugsweise 0,5 bis 95 Gewichtsprozent der Gesamtmischung.

In Übereinstimmung mit der Erfindung werden die Wirkstoffe in jeder geeigneten Formulierung angewandt unter der Voraussetzung, dass die Ausbildung bzw. Aufrechterhaltung von ausreichenden Wirkstoffspiegeln gewährleistet ist. Das kann beispielsweise durch orale oder parenterale Gabe in geeigneten Dosen erreicht werden. Vorteilhafterweise liegt die pharmazeutische Zubereitung der Wirkstoffe in Form von Einheitsdosen vor, die auf die gewünschte Verabreichung abgestimmt sind. Eine Einheitsdosis kann zum Beispiel eine Tablette, ein Dragée, eine Kapsel, ein Suppositorium oder eine gemessene Volumenmenge eines Pulvers, eines Granulates, einer Lösung, einer Emulsion oder einer Suspension sein.

Unter «Einheitsdosis» im Sinne der vorliegenden Erfindung wird eine physikalisch bestimmte Einheit, die eine individuelle Menge des aktiven Bestandteils in Kombination mit einem pharmazeutischen Trägerstoff enthält, verstanden, deren Wirkstoffgehalt einem Bruchteil oder einem Vielfachen einer therapeutischen Einzeldosis entspricht. Eine Einzeldosis enthält vorzugsweise die Menge Wirkstoff, die bei einer Applikation verabreicht wird und die gewöhnlich einer ganzen, einer halben, einer drittel oder einer viertel Tagesdosis entspricht. Wenn für eine einzelne therapeutische Verabreichung nur ein Bruchteil, wie die Hälfte oder ein Viertel, der Einheitsdosis benötigt wird, ist die Einheitsdosis vorteilhafterweise teilbar, z.B. in Form einer Tablette mit Bruchkerbe.

Die pharmazeutischen Zubereitungen gemäss der Erfindung können, wenn sie in Einheitsdosen vorliegen und für die Applikation z.B. am Menschen bestimmt sind, etwa 0,1 bis 500 mg, vorteilhafterweise 10 bis 200 mg und insbesondere 50 bis 150 mg Wirkstoff enthalten.

Im allgemeinen erweist es sich in der Humanmedizin als vorteilhaft, den oder die Wirkstoffe bei parenteraler Gabe in einer Tagesdosis von etwa 0,1 bis etwa 5, vorzugsweise 1 bis 3 mg/kg Körpergewicht, gegebenenfalls in Form mehrerer, vorzugsweise 1 bis 3 Einzelgaben zur Erzielung der gewünschten Ergebnisse zu verabreichen. Eine Einzelgabe enthält den oder die Wirkstoffe in Mengen von etwa 0,1 bis etwa 5, vorzugsweise 1 bis 3 mg/kg Körpergewicht. Bei einer oralen Behandlung können ähnliche Dosierungen zur Anwendung kommen.

Die therapeutische Verabreichung der pharmazeutischen Zubereitung kann 1 bis 4 mal am Tage zu festgelegten oder variierenden Zeitpunkten erfolgen, z.B. jeweils nach den Mahlzeiten und/oder am Abend. Es kann jedoch erforderlich sein, von den genannten Dosierungen abzuweichen, und zwar in Abhängigkeit von der Art, dem Körpergewicht und dem Alter des zu behandelnden Individuums, der Art und der Schwere der Erkrankung, der Art der Zubereitung und der Applikation des Arzneimittels sowie dem Zeitraum bzw. Intervall, innerhalb welchem die Verabreichung erfolgt. So kann es in einigen Fällen ausreichend sein, mit weniger als der oben genannten Menge Wirkstoff auszukommen, während in anderen Fällen die oben angeführte Wirkstoffmenge überschritten werden muss. Wie bei der internistischen Tumortherapie üblich, kann zur Reduzierung des Nebenwirkungsrisikos die Behandlung mit den erfindungsgemässen Arzneimittel kombiniert werden mit der Verabreichung anderer Cytostatica mit unterschiedlichen Wirkungsspektren. Es kann auch zweckmässig sein, die Behandlung nach dem Prinzip der zyklischen Cytostaticatherapie durchzuführen. Hierbei wird nach jeder Behandlung eine Erholungsphase eingelegt. Man macht sich dabei die Erfahrung zunutze, dass gesundes Gewebe der meisten Organe schneller regeneriert als malignes Gewebe.

Die Festlegung der jeweils erforderlichen optimalen Dosierung und Applikationsart der Wirkstoffe kann durch jeden Fachmann aufgrund seines Fachwissens leicht erfolgen.

Die pharmazeutischen Zubereitungen bestehen in der Regel aus den Komplexverbindungen und nichttoxischen, pharmazeutisch verträglichen Arzneimittelträger, die als Zumischung oder Verdünnungsmittel in fester, halbfester oder flüssiger Form oder als Umhüllungsmittel, beispielsweise in Form einer Kapsel, eines Tablettenüberzugs, eines Beutels oder eines anderen Behältnisses, für den therapeutisch aktiven Bestandteil in Anwendung kommen. Ein Trägerstoff kann z.B. als Vermittler für die Arzneimittelaufnahme durch den Körper, als Formulierungshilfsmittel, als Süssungsmittel, als Geschmackskorrigens, als Farbstoff oder als Konservierungsmittel dienen.

Zur oralen Anwendung können z.B. Tabletten, Dragées, harte und weiche Kapseln, z.B. aus Gelatine, dispergierbare Pulver, Granulate, wässrige und ölige Suspensionen, Emulsionen, Lösungen oder Sirupe kommen.

Tabletten können inerte Verdünnungsmittel, z.B. Calciumcarbonat, Calciumphosphat, Natriumphosphat oder Lactose; Granulierungs- und Verteilungsmittel, z.B. Maisstärke oder Alginate; Bindemittel, z.B. Stärke, Gelatine oder Akaziengummi; und Gleitmittel, z.B. Aluminium- oder Magnesiumstearat, Talkum oder Silikonöl, enthalten. Sie können zusätzlich mit einem Überzug versehen sein, der auch so beschaffen sein kann, dass er eine verzögerte Auflösung und Resorption des Arzneimittels im Gastrointestinaltrakt bewirkt, so dass z.B. eine bessere Verträglichkeit, Protrahierung oder eine Retardierung erreicht wird. Gelatinekapseln können den Arzneistoff vermischt mit einem festen z.B. Calciumcarbonat oder Kaolin, oder einem öligen z.B. Oliven-, Erdnuss- oder Paraffinöl, Verdünnungsmittel enthalten.

Wässrige Suspensionen können Suspendiermittel, z.B. Natriumcarboxymethylcellulose, Methylcellulose, Hydroxypropylcellulose, Natriumalginat, Polyvinylpyrrolidon, Traganthgummi oder Akaziengummi; Dispergier- und Benetzungsmittel, z.B. Polyoxyethylenstearat, Heptadecaethylenoxycetanol, Polyoxyethylensorbitanfettsäureester wie z.B. Polyoxyethylensorbitanmonooleat, sowie Lecithin; Konservierungsmittel, z.B. Methyl- oder Propylhydroxybenzoate; Geschmacksmittel; Süssungsmittel, z.B. Saccharose, Lactose, Natriumcyclamat, Dextrose, Invertzuckersirup, enthalten.

Ölige Suspensionen können z.B. Erdnuss-, Oliven-, Sesam-, Kokos- oder Paraffinöl und Verdikkungsmittel, wie z.B. Bienenwachs, Hartparaffin, oder Cetylalkohol, enthalten; ferner Süssungsmittel, Geschmacksmittel und Antioxidantien.

In Wasser dispergierbare Pulver und Granulate können die Komplexverbindungen in Mischung mit Dispergier-, Benetzungs- und Suspendiermitteln, z.B. den oben genannten, sowie Süssungsmitteln, Geschmacksmitteln und Farbstoffen enthalten.

Emulsionen können z.B. Oliven-, Erdnuss- und Paraffinöl neben Emulgiermitteln, wie z.B. Akaziengummi, Traganthgummi, Phosphatiden, Sorbitanmonooleat, Polyoxyethylensorbitanmonooleat, und Süssungs- und Geschmacksmittel enthalten.

Zur parenteralen Anwendung der Arzneistoffe dienen steril injizierbare wässrige Suspensionen, isotonische Salzlösungen oder sonstige Lösungen, die Dispergier- oder Benetzungsmittel und/oder pharmakologisch verträgliche Verdünnungsmittel, z.B. Propylen- oder Butylenglykol, und/oder Lösungsvermittler, z.B. Tweene®, Cremophore® oder Polyvinylpyrrolidon, enthalten.

Besonders vorteilhaft ist es, Lösungen der Komplexverbindungen in einem wasserfreien organischen Lösungsmittel mit Lösungen von hydrophilen Polymeren, wie beispielsweise Polyvinylpyrrolidonen (PVP) oder Polyoxyethylensorbitanfettsäureestern (Tween®) oder insbesondere Glycerin-Polyethylenglykolricinoleat (Cremophor®EL) zu vermischen und die nach Abziehen des bzw. der Lösungsmittel zurückbleibenden Rückstände als wässrige Lösungen zu verabreichen. Als organische Lösungsmittel kommen beispielsweise Chloroform oder Methylenchlorid in Frage, die vor der Verwendung auf übliche Weise wasserfrei gemacht werden. Es hat sich als zweckmässig erwiesen, die hydrophilen Polymeren in einem 5- bis 50-, vorzugsweise 10- bis 35fachen gewichtsmässigen Überschuss gegenüber der Komplexverbindung anzuwenden. Es ist auch möglich, die Polymeren als solche in Lösungen der Komplexverbindungen einzutragen. Den nach Abziehen des bzw. der Lösungsmittel verbleibenden Rückstand befreit man zweckmässigerweise im Hochvakuum möglichst weitgehend von Lösungsmittelresten. Man erhält je nach Art und Menge des verwendeten hydrophilen Polymeren feste kristallartige oder glasartige oder auch flüssige oder klebrige Rückstände. Letztere lassen sich in der Regel durch Abkühlung in feste, meist wachsartige Produkte überführen. Im Sinne der vorliegenden Erfindung sollen diese Rückstände als Kopräzipitate bezeichnet werden. Günstig auf das Löseverhalten der Kopräzipitate wirkt es sich aus, wenn man bei deren Herstellung zu dem Gemisch der Lösungen der Komplexverbindung und des hydrophilen Polymeren noch Dispergier- oder Benetzungsmittel, wie Propylen- oder Butylenglykol, vorzugsweise Propylenglykol, zugibt.

Die Herstellung der wässrigen Lösungen der Kopräzipitate erfolgt durch Behandeln der Kopräzipitate mit Wasser. PVP-Kopräzipitate gehen in der Regel bereits bei Raumtemperatur in Lösung. Kopräzipitate mit Polyoxyethylensorbitanfettsäureestern oder Glycerin-Polyethylenglykolricinooleat sind vorteilhaft in Lösung zu bringen, wenn man das Kopräzipitat und das Wasser vor dem Zusammengeben auf 25 bis 60°C, vorzugsweise 30 bis 40°C erwärmt.

Besonders vorteilhaft ist es, die Kopräzipitate anstatt in Wasser in physiologischer Kochsalzlösung aufzulösen.

Kopräzipitate, erhalten aus den Komplexverbindungen der weiter vorn angegebenen allgemeinen Formel I, worin M, $R^1$, $R^2$, $R^3$, $R^4$, X, m und n die dort angegebenen Bedeutungen haben, und hydrophilen Polymeren, Verfahren zur Herstellung dieser Kopräzipitate, sowie wässrige Lösungen enthaltend diese Kopräzipitate, sind weitere bevorzugte Gegenstände der vorliegenden Erfindung.

Der Wirkstoff kann gegebenenfalls mit einem oder mehreren der angegebenen Träger- oder Zusatzstoffe auch in mikroverkapselter Form formuliert werden.

## HERSTELLUNGSBEISPIELE

1. Diethoxybis-(1-phenyl-1,3-butandionato)-titan(IV)

Zu 11,4 g (0,05 Mol) Titantetraethoxid werden unter Feuchtigkeitsausschluss 15,8 g (0,097 Mol) Benzoylaceton in 200 ml trockenem n-Hexan schnell zugetropft. Das Reaktionsgemisch wird zwei Stunden am Rückfluss in einer trockenen Stickstoffatmosphäre erhitzt. Das ausfallende farblose Produkt wird abgesaugt, in siedendem Hexan aufgeschlämmt, nochmals abgesaugt und am Hochvakuum von Lösungsmittelresten befreit. Schmelzpunkt: 110°C.

Eine weitere Herstellungsmethode ist zu finden bei O.M. Puri, R.C. Mehrotra, J. Ind. Chem. Soc. 39(8), 499 (1962).

2. Diethoxybis-(1-phenyl-2,4-pentandionato)-titan(IV)

In trockener Stickstoffatmosphäre werden zu 3,7 g (0,021 Mol) 1-Phenyl-2,4-pentandion unter heftigem Rühren 2,2 ml (0,0105 Mol) Titantetraethoxid zugegeben. Aus der gelben Reaktionsmischung werden die flüchtigen Anteile zunächst am Wasserstrahlpumpenvakuum und dann im Ölpumpenvakuum abgezogen. Es bleiben 5,1 g eines roten Öls zurück. Ausbeute: quantitativ.

Analyse:

Ber.: 63,93% C; 6,60% H; 9,81% Ti
Gef.: 63,44% C; 6,71% H; 10,14% Ti.

Die Darstellung von 1-Phenyl-2,4-pentandion erfolgt nach R. Levine et al., J. Am. Chem. Soc. 67, 1510 (1945).

3. Diethoxybis-(4,4-dimethyl-1-phenyl-1,3-pentandionato)-titan(IV)

5,28 g (0,0097 Mol) der Titelverbindung erhält man als rötlichgelbes Öl in quantitativer Ausbeute in analoger Vorgehensweise wie bei Beispiel 2 aus 3,95 g (0,0194 Mol) 4,4-Dimethyl-1-phenyl-1,3-pentandion und 2,21 g (0,0097 Mol) Titantetraethoxid.

Analyse:

Ber.: 66,17% C; 7,40% H; 8,80 Ti
Gef.: 66,44% C; 7,57% H; 8,34 Ti.

Die Darstellung von 4,4-Dimethyl-1-phenyl-1,3-pentandion ist beschrieben bei R. Levine et al., J. Am. Chem. Soc. 73, 5614 (1951).

4. Diethoxybis-(1-phenyl-1,3-hexandionato)-titan(IV)

5 g (0,0097 Mol) der Titelverbindung erhält man als gelbes Öl in quantitativer Ausbeute in analoger Vorgehensweise wie bei Beispiel 2 aus 3,69 g (0,0194 Mol) 1-Phenyl-1,3-hexandion und 2,21 g (0,0097 Mol) Titantetraethoxid.

Analyse:

Ber.: 65,12% C; 7,02% H; 9,27% Ti
Gef.: 65,38% C; 7,30% H; 9,30% Ti.

5. Diethoxybis-(1-phenyl-1,3-pentandionato)-titan(IV)

4,73 g (0,0097 Mol) der Titelverbindung erhält man als gelbes Öl in quantitativer Ausbeute in analoger Vorgehensweise wie bei Beispiel 2 aus 3,42 g (0,00194 Mol) 1-Phenyl-1,3-pentandion und 2,21 g (0,0097 Mol) Titantetraethoxid.

Analyse:

Ber.: 63,94% C; 6,60% H; 9,80% Ti
Gef.: 63,92% C; 6,61% H; 9,67% Ti.

6. Di-(n-propoxy)-bis-(1-phenyl-1,3-butandionato)-titan(IV)

Zu einer benzolischen Lösung von 7,1 g (0,025 Mol) Titantetra-n-propoxid werden in trokkener Stickstoffatmosphäre 8,11 g (0,05 Mol) Benzoylaceton in Benzol gegeben. Nach 3 Stunden Kochen am Rückfluss wird das Benzol abdestilliert, der Rückstand mit Diethylether aufgenommen und durch Zugabe von n-Hexan die Titelverbindung ausgefällt und im Hochvakuum getrocknet.

Ausbeute: 11,5 g (94% d.Th.); Schmelzpunkt: 133°C.

Analyse:

Ber.: 63,9% C; 6,6% H; 9,8% Ti
Gef.: 63,5% C; 6,5% H; 10,3% Ti.

7. Bis-(natrium-2-sulfonatoethanolato)-bis-(1-phenyl-1,3-butandionato)-titan(IV)

2,96 g (0,022 Mol) Natriumsalz der 2-Hydroxyethansulfonsäure werden in trockener Stickstoffatmosphäre mit 4,41 g (0,01 Mol) Dichloro-bis-(1-phenyl-1,3-butandionato)-titan(IV) in 250 ml Benzol unter Rückfluss 3 Tage gerührt. Nach Beendigung der Chlorwasserstoffentwicklung wird aus der gelben Lösung mit Petrolether (Kochpunkt 60 bis 70°C) ein gelbes Produkt gefällt und im Hochvakuum getrocknet.

Ausbeute: 6,4 g (96% d.Th.); Schmelzpunkt: 184°C.

Analyse:

Ber.: 43,4% C; 3,9% H
Gef.: 43,4% C; 4,0% H.

8. Di-(2-propanolato)-bis-(1-phenyl-1,3-butandionato)-titan(IV)

In trockener Stickstoffatmosphäre werden zu 3,55 g (0,0125 Mol) Titantetraisopropoxid in Benzol 4,05 g (0,025 Mol) Benzoylaceton in Benzol gegeben. Das Reaktionsgemisch wird 2 bis 3 Stunden am Rückfluss erhitzt und nach teilweisem Abdestillieren des Benzols mit heissem n-Hexan versetzt. Beim Abkühlen wird gerührt und anschliessend wird das ausgeschiedene gelbe Produkt abgesaugt und am Hochvakuum getrocknet (Literatur siehe Beispiel 1).

Ausbeute: 5,6 g (93% d.Th.); Schmelzpunkt: 89°C.

Analyse:

Ber.: 63,9% C; 6,6% H; 9,8% Ti
Gef.: 63,9% C; 6,6% H; 9,6% Ti.

9. Chloro-(2-hydroxyethanolato)-bis-(1-phenyl-1,3-butandionato)-titan(IV)

Zu 4,41 g (0,01 Mol) Dichlorobis-(1-phenyl-1,3-butandionato)-titan(IV) in 80 ml Benzol werden in trockener Stickstoffatmosphäre unter Erwärmen 0,62 g (0,01 Mol) Ethylenglykol, gelöst in 40 ml Benzol zugetropft. Nach Beendigung der Chlorwasserstoffentwicklung (etwa 3 Stunden) wird zur Trockne eingeengt und der Rückstand mit Methylenchlorid aufgenommen. Durch Zugabe von n-Hexan wird das gelbe Produkt ausgefällt und am Hochvakuum getrocknet.

Ausbeute: 4,25 g (91% d.Th.); Schmelzpunkt: 73°C.

Analyse:

Ber.: 56,6% C; 5,0% H; 10,2% Ti
Gef.: 57,1% C; 5,3% H; 10,8% Ti.

10. Chloro-(2-hydroxypropanolato)-bis-(1-phenyl-1,3-butandionato)-titan(IV)

Zu 4,41 g (0,01 Mol) Dichlorobis-(1-phenyl-1,3-butandionato)-titan(IV) in 80 ml Benzol werden in trockener Stickstoffatmosphäre unter Erwärmen 0,76 g (0,01 Mol) 1,2-Propandiol in 40 ml Benzol zugetropft. Nach Beendigung der Chlorwasserstoffentwicklung wird das gelbe Produkt durch Zugabe von n-Hexan gefällt. Nach einmaligem Umfällen aus Methylenchlorid mittels n-Hexan wird das gelbe Pulver am Hochvakuum getrocknet.

Ausbeute: 4,6 g (96% d.Th.); Schmelzpunkt: 78°C.

Analyse:

| | | | |
|---|---|---|---|
| Ber.: | 57,5% C; | 5,2% H; | 10,0% Ti |
| Gef.: | 57,5% C; | 5,6% H; | 10,6% Ti. |

11. Chloro-(tert.-butanolato)-bis-(1-phenyl-1,3-butandionato)-titan(IV)

In trockener Stickstoffatmosphäre werden zu 4,41 g (0,01 Mol) Dichloro-bis-(phenyl-1,3-butandionato)-titan(IV), aufgeschlämmt in 70 ml Methylenchlorid 0,74 g (0,01 Mol) tert.-Butanol, gelöst in 20 ml Methylenchlorid zugetropft. Es wird solange am Rückfluss gekocht, bis kein Chlorwasserstoff mehr entweicht. Es wird vorsichtig mit Petrolether (Kochpunkt 60 bis 70°C) versetzt, bis ein braunes Öl ausfällt. Nach dem Absetzen des Öls wird die überstehende gelbe Lösung dekantiert und soweit eingeengt, bis ein gelbes Produkt ausfällt. Dieses wird abgesaugt und mit viel Petrolether gewaschen.

Ausbeute: 1,9 g (40% d.Th.); Schmelzpunkt: 70–75°C.

Analyse:

| | | | |
|---|---|---|---|
| Ber.: | 60,2% C; | 5,7% H; | 10,0% Ti |
| Gef.: | 60,3% C; | 5,9% H; | 10,0% Ti. |

12. Chloro-(2,3-dimethyl-2,3-butandiolato)-bis-(1-phenyl-1,3-butandionato)-titan(IV)

In trockener Stickstoffatmosphäre werden zu einer Aufschlämmung von 4,41 g (0,01 Mol) Dichlorobis-(1-phenyl-1,3-butandionato)-titan(IV) in 100 ml trockenem Methylenchlorid 1,18 g (0,01 Mol) 2,3 Dimethyl-2,3-butandiol, gelöst in 30 ml Methylenchlorid, zugetropft. Es wird solange am Rückfluss gekocht, bis kein Chlorwasserstoff mehr entwickelt wird. Die Lösung wird bis zur beginnenden Trübung mit Petrolether (Kochpunkt 60 bis 70°C) versetzt. Nach mehrstündigem Stehen bei −18°C wird der gelbe Niederschlag abfiltriert und mit Petrolether gewaschen.

Ausbeute: 2,1 g (40% d.Th.); Schmelzpunkt: 80–90°C.

Analyse:

| | | | |
|---|---|---|---|
| Ber.: | 59,7% C; | 6,0% H; | 9,2% Ti |
| Gef.: | 58,9% C; | 6,3% H; | 9,2% Ti. |

13. Chloro-mentholato-bis-(1-phenyl-1,3-butandionato)-titan(IV)

In trockener Stickstoffatmosphäre werden zu einer Aufschlämmung von 4,41 g (0,01 Mol) Dichlorobis-(1-phenyl-1,3-butandionato)-titan(IV) in 70 ml Methylenchlorid 1,56 g (0,01 Mol) Menthol, gelöst in 30 ml Methylenchlorid rasch zugetropft. Es wird bis zur Beendigung der Chlorwasserstoffentwicklung am Rückfluss gekocht. Nach Abkühlen der Mischung auf Zimmertemperatur wird durch vorsichtige Zugabe von Petrolether (Kochpunkt 60 bis 70°C) das strahlend gelbe Produkt ausgefällt. Dieses wird abgesaugt und mit Petrolether (Kochpunkt 60 bis 70°C) gewaschen.

Ausbeute: 2,24 g (40% d.Th.); Schmelzpunkt: 95°C.

Analyse:

| | | | |
|---|---|---|---|
| Ber.: | 64,2% C; | 6,7% H; | 8,5% Ti |
| Gef.: | 64,2% C; | 6,8% H; | 8,6% Ti. |

14. Diethoxybis-(1-phenyl-1,3-butandionato)-hafnium(IV)

Zu einer Aufschlämmung von 10 g Hafniumtetrachlorid in 400 ml trockenem Ether werden unter einem Stickstoffstrom und unter Sieden am Rückfluss 30,4 g Benzoylaceton, gelöst in 200 ml trockenem Ether, zugetropft. Nach 24stündigem Kochen am Rückfluss wird der gebildete Niederschlag abfiltriert und zweimal mit je 100 ml Ether gewaschen und in ungefähr 600 ml Chloroform gelöst. Nach dem Einengen bis zum Auftreten einer Trübung wird filtriert und die Lösung bei −5°C stehengelassen. Der farblose Niederschlag wird aus Chloroform umkristallisiert. Man erhält 6 g (33,7% d.Th.) Dichlorobis-(1-phenyl-1,3-butandionato)-hafnium(IV) vom Schmelzpunkt 231–232°C (rote Schmelze).

In trockener Stickstoffatmosphäre werden 5,71 g (0,01 Mol) Dichloro-bis-(1-phenyl-1,3-butandionato)-hafnium(IV) und 1,2 g (0,026 Mol) absolutes Ethanol in 100 ml Benzol unter Einleiten von trockenem Ammoniak gerührt. Nach Abklingen der exothermen Reaktion wird vier Stunden am Rückfluss erhitzt. Danach wird drei Tage lang bei Raumtemperatur trockenes Ammoniak eingeleitet. Nach Abzentrifugieren des entstandenen Ammoniumchlorids wird die Lösung zur Trockne eingeengt. Der Rückstand wird am Hochvakuum getrocknet, wobei bei 90°C Badtemperatur weisse Kristalle absublimieren. Die Titelverbindung bleibt zurück.

Ausbeute: 4,41 g (74,7% d.Th.); Schmelzpunkt: 98°C (Zusammensintern bei 87–97°C).

Analyse:

| | | |
|---|---|---|
| Ber.: | 48,78% C; | 4,77% H |
| Gef.: | 50,09% C; | 4,33% H. |

15. Bis-(2-diethylaminoethoxy)-bis-(1-phenyl-1,3-butandionato)-hafnium(IV)hydrochlorid

Unter trockener Stickstoffatmosphäre werden 5,71 g (0,01 Mol) Dichloro-bis-(1-phenyl-1,3-bu-

tandionato)-hafnium(IV) und 2,34 g (0,02 Mol) frisch destilliertes Diethylaminoethanol in 70 ml trockenem Methylenchlorid in einem fest verschlossenen Kolben zwei Tage gerührt. Das Reaktionsgemisch wird am Rotationsverdampfer eingeengt und der Rückstand am Hochvakuum getrocknet.

Ausbeute: 5,08 g (76% d.Th.); Schmelzpunkt: 135°C (Zusammensintern ab 125°C).

16. Diethoxybis-(1-phenyl-1,3-butandionato)-zirkon(IV)

Unter trockener Stickstoffatmosphäre werden 3,87 g (0,008 Mol) Dichlorobis-(1-phenyl-1,3-butandionato)-zirkonium(IV) in 50 ml trockenem Methylenchlorid aufgeschlämmt und mit einer Lösung von 1,71 g (0,025 Mol) Natriumethanolat in 50 ml absolutem Ethanol versetzt. Nach einem Tag Kochen am Rückfluss wird drei Tage lang trockenes Ammoniak eingeleitet. Nach Abfiltrieren des Niederschlags wird das Filtrat durch Zentrifugieren von Resten des Niederschlags befreit. Die klare Lösung wird am Rotationsverdampfer zur Trockne eingeengt und der Rückstand am Hochvakuum getrocknet, wobei bei 90°C Badtemperatur weisse Kristalle absublimieren. Die Titelverbindung bleibt zurück.

Ausbeute: 5,8 g (72% d.Th.); Schmelzpunkt: 122–124°C.

17. Bis-(1-phenyl-1,3-butandionato)-di-(n-propanolato)-zirkon(IV)

10,38 g (0,05 Mol) Tetra-n-propoxyzirkon(IV) und 16,2 g (0,01 Mol) Benzoylaceton werden in 100 ml frisch destilliertem Chlorbenzol gelöst. Das Chlorbenzol/Propanol-Azeotrop wird abdestilliert, bis die Siedetemperatur 132°C erreicht ist. Am Rotationsverdampfer wird sodann bis zur Trockene eingeengt. Der Rückstand wird vier Stunden bei 80°C im Hochvakuum und dann über Nacht bei Raumtemperatur getrocknet.

Ausbeute: 23,4 g (88% d.Th.); Schmelzpunkt: −6°C.

Analyse:

Ber.: 58,7% C; 6,1% H
Gef.: 60,8% C; 5,5% H.

18. Di-(n-butanolato)-bis-(1-phenyl-1,3-butandionato)-zirkon(IV)

Analog zu Beispiel 17 werden 28,62 g (0,0625 Mol) Tetra-(n-butoxy)-zirkon(IV) · Butanol mit 20,65 g (0,125 Mol) Benzoylaceton in 100 ml Chlorbenzol umgesetzt. Der Rückstand wird im Hochvakuum fünf Stunden bei 70°C und anschliessend zehn Stunden bei Raumtemperatur getrocknet.

Ausbeute: 28,7 g (82% d.Th.); Schmelzpunkt: −2°C.

Analyse:

Ber.: 60,1% C; 6,5% H
Gef.: 58,3% C; 6,3% H.

19. Bis-(tridecanolato)-bis-(1-phenyl-1,3-butandionato)-titan(IV)

Unter trockener Stickstoffatmosphäre wird zu einer Lösung von 4,6 g (0,01 Mol) Diethoxybis-(1-phenyl-1,3-butandionato)-titan(IV) in 100 ml Methylenchlorid eine Lösung von 4,0 g (0,02 Mol) 1-Tridecanol (Tridecylalkohol, $CH_3(CH_2)_{12}OH$) in 50 ml Methylenchlorid schnell zugetropft. Es wird zwei Stunden bei Raumtemperatur gerührt und anschliessend am Rotationsverdampfer eingeengt. Das resultierende rote Öl wird am Hochvakuum von flüchtigen Stoffen befreit. Es zersetzt sich ab 250°C.

Ausbeute: 7,68 g (100% d.Th.); Kochpunkt (26,6 Pa): 140°C.

Analyse:

Ber.: 70,2% C; 9,5% H; 6,1% Ti
Gef.: 69,5% C; 9,4% H; 6,0% Ti.

20. Diethoxybis-[1-(3,4-dimethoxyphenyl)-1,3-butandionato]-titan(IV)

In trockener Stickstoffatmosphäre werden zu 2,28 g (0,01 Mol) Titantetraethoxid, gelöst in 50 ml Methylchlorid, 4,44 g (0,02 Mol) 1-(3,4-Dimethoxyphenyl)-1,3-butandion, gelöst in 20 ml Methylenchlorid, schnell zugetropft. Die klare gelbe Lösung wird eineinhalb Stunden bei Raumtemperatur gerührt, anschliessend zur Trockne eingeengt. Der gelbe Rückstand wird am Hochvakuum getrocknet.

Ausbeute: quantitativ; Schmelzpunkt: 98–105°C.

Analyse:

Ber.: 57,9% C; 6,3% H
Gef.: 58,2% C; 6,3% H.

21. Diethoxybis-(3-phenyl-2,4-pentandionato)-titan(IV)

2,3 g (0,01 Mol) Tetraethoxytitan(IV) und 3,5 g (0,02 Mol) 3-Phenyl-2,4-pentandion [Herstellung nach J.T. Adams, C.R. Hauser, J. Am. Chem. Soc. 67, 284 (1945)] in 100 ml Benzol werden in trockener Stickstoffatmosphäre am Rückfluss erhitzt. Das Ethanol/Benzol-Azeotrop wird abdestilliert, bis das Gemisch eine Siedetemperatur von 80°C erreicht hat. Die rotbraune klare Lösung wird zur Trockne eingeengt. Die rötlich-gelbe Masse wird aus Benzol umkristallisiert und am Hochvakuum getrocknet.

Ausbeute: 3,2 g (65% d.Th.); Schmelzpunkt: 103–112°C (Zersetzung).

Analyse:

Ber.: 63,93% C; 6,60% H; 9,91 Ti
Gef.: 63,78% C; 6,77% H; 9,72% Ti.

22. Kopräzipitat Diethoxy-bis-(1-phenyl-1,3-butandionato)-titan(IV)/Cremophor®EL 1:10

In trockener Stickstoffatmosphäre werden Lösungen von 1 g Diethoxy-bis-(1-phenyl-1,3-bu-

tandionato)-titan(IV) und 10 g getrocknetem Cremophor®EL (Firma BASF) in trockenem reinem Ethanol bereitet. Nach Vereinigen der beiden Lösungen wird das Lösungsmittel unter vermindertem Druck abgezogen. Das zurückbleibende gelbliche Kopräzipitat wird anschliessend für 24 Stunden unter Hochvakuum gehalten. Das Kopräzipitat wird in Wasser oder physiologischer Kochsalzlösung aufgelöst.

Eine Lösung des Kopräzipitats in physiologischer Kochsalzlösung wird besonders vorteilhaft bereitet, indem man das in einer Spritze auf 40°C vorgewärmte Kopräzipitat unter Rühren in eine ebenfalls auf 40°C vorgewärmte physiologische Kochsalzlösung (0,9%ige wässrige Natriumchloridlösung) einspritzt.

Pharmakologie

A. Tumormodelle

1. Sarkom 180-Tumormodell (intraperitoneal)

Ca. 6 Wochen alten 18 bis 20 g schweren weiblichen NMRI-Mäusen werden je ca. $10^6$ Sarkom 180-Tumorzellen in 0,2 ml physiologischer Kochsalzlösung intraperitoneal (i.p.) übertragen. Der Tumor wird auf dem selben Mäusestamm in Passage gehalten. Die Tumorzellen werden frisch getöteten Tieren unmittelbar vor der Transplantation entnommen. Beim Überimpfen werden die Tiere randomisiert. Pro Dosierung werden 6 Mäuse verwendet. Die Anzahl der Kontrollgruppen (unbehandelte Tiere) wird so gewählt, dass sie in etwa der Quadratzahl aus der Gesamtzahl der Gruppen entspricht. Die Substanzen werden als Suspensionen in üblichen Lösungsvermittlern, wie beispielsweise Tween® (Polyoxyethylenderivate von Sorbitanestern), jeweils 24 Stunden nach der Transplantation intraperitoneal gespritzt.

2. Sarkom 180-Tumormodell (subkutan)

NMRI-Mäusen wie unter 1. werden je ca. $20 \cdot 10^6$ Sarkom 180-Tumorzellen in 0,2 ml physiologischer Kochsalzlösung subkutan (s.c.) übertragen. Bei einem medianen Tumorgewicht von 0,5 g, was unter den gegebenen Versuchsbedingungen nach etwa 8 Tagen erreicht wird, wird mit der Therapie begonnen. Die Metallkomplexe werden als Lösungen von Kopräzipitaten in physiologischer Kochsalzlösung zweimal wöchentlich in die Schwanzvene verabreicht.

Das Tumorgewicht wird während des Versuchs auf die übliche Weise durch Palpieren und Vergleichen mit standardisierten Knetkugeln geschätzt.

3. Ehrlich Ascites-Tumormodell

NMRI-Mäusen wie unter 1. werden am Tag 0 je $10^6$ Ehrlich Ascites-Tumorzellen in 0,2 ml physiologischer Kochsalzlösung intraperitoneal übertragen. Am Tag 1 werden die Metallkomplexe als Lösungen von Kopräzipiten in physiologischer Kochsalzlösung intraperitoneal verabreicht. Am Tag 10 werden die Tiere getötet und die Ehrlich Ascites-Tumorzellen ausgezählt. Es werden drei Tiere pro Gruppe verwendet.

B. Versuchsergebnisse

In der nachfolgenden Tabelle I sind Ergebnisse aus dem unter Punkt A 1. beschriebenen Sarkom 180-Tumormodell zusammengestellt. Die angegebene Dosis wurde einmalig zu Beginn des Versuchs wie angegeben appliziert. Der in Prozent angegebene Faktor T/C bedeutet die prozentuale Verlängerung der medianen Überlebenszeit der behandelten Tiere im Vergleich zur medianen Überlebenszeit der unbehandelten Kontrolltiere.

Der Versuch wird abgebrochen, sobald die mediane Überlebenszeit T/C der behandelten Tiere 300% der medianen Überlebenszeit der unbehandelten Tiere erreicht hat. Zur Berechnung der medianen Überlebenszeit werden die bei Versuchsende noch lebenden Tiere als bei Versuchsende gestorben berücksichtigt. Die Vergleichsverbindung Cisplatin wirkt in einem Dosisbereich von etwa 8 bis 10 mg/kg therapeutisch und die Tiere werden zum grossen Teil geheilt. Die mediane Überlebenszeit der mit Cisplatin behandelten Tiere wird bereits bei einer Dosis von etwa 20 mg/kg kleiner als die der unbehandelten Kontrolltiere.

Tabelle I

| $[R^1(CH_2)_mC(O)CR^3C(O)R^2]_2M(OR^4)_{2-n}X_n$ | | | | | | | | Dosis [mg/kg] | T/C [%] |
|---|---|---|---|---|---|---|---|---|---|
| $R^1$ | $R^2$ | $R^3$ | $R^4$ | M | X | m | n | | |
| 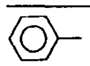 | $CH_3$ | H | $C_2H_5$ | Ti | - | 0 | 0 | 18<br>92<br>198 | 300<br>279<br>300 |
| 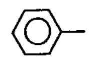 | $CH_3$ | H | n-$C_4H_9$ | Ti | - | 0 | 0 | 222 | 139 |
| 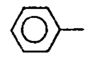 | $CH_3$ | H | n-$C_3H_7$ | Ti | - | 0 | 0 | 210 | 133 |
| 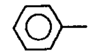 | $CH_3$ | H | i-$C_3H_7$ | Ti | - | 0 | 0 | 20<br>98 | 283<br>195 |
| 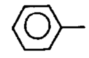 | $CH_3$ | H | t-$C_4H_9$ | Ti | Cl | 0 | 1 | 96<br>206 | 184<br>204 |
| 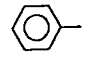 | $CH_3$ | H | $NaSO_3CH_2CH_2$ | Ti | - | 0 | 0 | 133<br>286 | 144<br>224 |

Tabelle I (Fortsetzung)

| $[R^1(CH_2)_mC(O)CR^3C(O)R^2]_2M(OR^4)_{2-n}X_n$ | | | | | | | | Dosis [mg/kg] | T/C [%] |
|---|---|---|---|---|---|---|---|---|---|
| $R^1$ | $R^2$ | $R^3$ | $R^4$ | M | X | m | n | | |
| 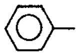 | n-$C_3H_7$ | H | $C_2H_5$ | Ti | – | 0 | 0 | 222 | 153 |
| 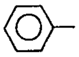 | $CH_3$ | H | $(CH_3)_2C(OH)C(CH_3)_2$ | Ti | Cl | 0 | 1 | 21<br>105 | 242<br>138 |
| 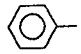 | $CH_3$ | H | $CH_3CH(OH)CH_2$ | Ti | Cl | 0 | 1 | 19<br>94<br>203 | 215<br>283<br>283 |
| 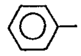 | $CH_3$ | H | $HOCH_2CH_2$ | Ti | Cl | 0 | 1 | 19<br>93<br>201 | 145<br>283<br>211 |
| 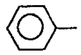 | $CH_3$ | H | $C_2H_5$ | Ti | – | 1 | 0 | 98<br>210 | 144<br>195 |
| $CH_3$ | $CH_3$ | 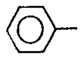 | $C_2H_5$ | Ti | – | 0 | 0 | 20<br>98<br>210 | 300<br>250<br>214 |
| 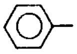 | 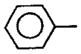 | H | $C_2H_5$ | Ti | – | 0 | 0 | 251 | 300 |
| 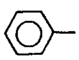 | $CH_3$ | H | $CH_3(CH_2)_3CH(C_2H_5)CH_2$ | Ti | – | 0 | 0 | 25<br>125<br>269 | 233<br>233<br>273 |
| 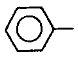 | $CH_3$ | H | 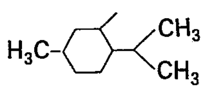 | Ti | Cl | 0 | 1 | 22<br>112<br>241 | 197<br>187<br>172 |
| 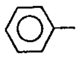 | $CH_3$ | H | $CH_3(CH_2)_{12}$ | Ti | – | 0 | 0 | 31<br>154 | 300<br>300 |
| 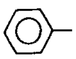 | $C_2H_5$ | H | $C_2H_5$ | Ti | – | 0 | 0 | 20<br>98<br>210 | 204<br>276<br>135 |
| t-$C_4H_9$ | $CH_3$ | H | $C_2H_5$ | Ti | – | 0 | 0 | 181 | 165 |

In der nachfolgenden Tabelle II sind die Ergebnisse zu dem unter Punkt A 2. beschriebenen Sarkom 180-Tumormodell angegeben. Man sieht einen deutlichen Rückgang des Tumorgewichts im Vergleich zu den Kontrolltieren.

Tabelle II

| Substanz[1)] | Anzahl der Tiere | Dosis [mg/kg] | Tumorgewicht [g] | | | | | | |
|---|---|---|---|---|---|---|---|---|---|
| | | | Tag 0 | Tag 8 | Tag 13 | Tag 16 | Tag 20 | Tag 23 | Tag 27 |
| Kontrolle | 12* | 0 | Tumortransplantation | 0,5 | 0,85 | 0,85 | 1,1 | 0,75 | 0,8 |
| $Ti(bzac)_2(OEt)_2$ | 6 | 5 | | 0,5 | 0,5 | 0,45 | 0,3 | 0,15 | 0 |
| T60 1:30 | 6 | 10 | | 0,65 | 0,5 | 0,6 | 0,55 | 0,35 | 0,5 |
| | 6 | 20 | | 0,5 | 0,4 | 0,35 | 0,25 | 0,15 | 0,05 |
| $Ti(bzac)_2(OEt)_2$ | 6 | 5 | | 0,65 | 1,0 | 0,8 | 0,85 | 1,05 | 0,75 |
| Cremophor® | 6 | 10 | | 0,5 | 0,5 | 0,35 | 0,2 | 0,1 | 0 |
| 1:10 | 6 | 20 | | 0,5 | 0,7 | 0,4 | 0,4 | 0,1 | 0 |
| $Ti(bzac)_2(OEt)_2$ | 6 | 5 | | 0,6 | 0,85 | 0,45 | 0,75 | 1,5 | 0,6 |
| PVP 30 BT | 6 | 10 | | 0,55 | 0,5 | 0,25 | 0,15 | 0 | 0 |
| 1:10 | 6 | 20 | | 0,8 | 0,7 | 0,25 | 0,3 | 0,3 | 0,15 |

* ab Tag 20 sind es 30 Tiere, da vorher 18 Kontrolltiere versehentlich nicht berücksichtigt wurden;

[1)] $Ti(bzac)_2(OEt)_2$ = Diethoxy-bis-(1-phenyl-1,3-butandionato)-titan(IV)

T60 = Tween®60;

PVP 30 BT = Polyvinylpyrrolidon mit mittlerem Molekulargewicht 30 000.

In der nachfolgenden Tabelle III sind die Ergebnisse zu dem unter Punkt A 3. beschriebenen Ehrlich Ascites-Tumormodell angegeben. Bei den behandelten Tieren beobachtet man eine bedeutend geringere Zahl von Ehrlich Ascites-Tumorzellen als bei den unbehandelten Kontrolltieren.

Tabelle III

| $[R^1(CH_2)_mC(O)CR^3C(O)R^2]_2M(OR^4)_{2-n}X_n$ | | | | | | | | Dosis [mg/kg] | Ehrlich Ascites-Zellen am Tag 10 |
|---|---|---|---|---|---|---|---|---|---|
| $R^1$ | $R^2$ | $R^3$ | $R^4$ | M | X | m | n | | |
| 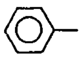 | $CH_3$ | H | $C_2H_5$ | Hf | – | 0 | 0 | 24<br>118<br>254 | 64,8·10⁷<br>33,9·10⁷<br>0 |
| 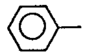 | $CH_3$ | H | $HCl\cdot(C_2H_5)_2NC_2H_4$ | Hf | – | 0 | 0 | 347 | 0 |
| | Kontrolle | | | | | | | 0 | 102,5·10⁷ |

**Patentansprüche für die Vertragsstaaten BE, CH, DE, FR, GB, IT, LI, LU, NL, SE**

1. Arzneimittel enthaltend einen oder mehrere Metallkomplexe der allgemeinen Formel I,

$$[R^1(CH_2)_mC(O)CR^3C(O)R^2]_2M(OR^4)_{2-n}X_n \quad (I),$$

worin
M Titan, Zirkon oder Hafnium,
$R^1$ Wasserstoff, $C_1$–$C_8$-Alkyl oder Phenyl, das durch Fluor, Chlor, Brom, Nitro, $C_1$-$C_4$-Alkyl, $C_1$–$C_4$-Alkoxy oder Trifluormethyl einfach oder mehrfach substituiert sein kann,
$R^2$ $C_1$–$C_8$-Alkyl oder Phenyl, das durch Fluor, Chlor, Brom, Nitro, $C_1$–$C_4$-Alkyl, $C_1$–$C_4$-Alkoxy oder Trifluormethyl einfach oder mehrfach substituiert sein kann,
$R^3$ Wasserstoff oder Phenyl,
$R^4$ $C_1$–$C_{18}$-Alkyl, das durch Hydroxy, $C_1$–$C_3$-Alkylamin oder Alkalisulfonatogruppen substituiert sein kann, oder $C_5$–$C_8$-Cycloalkyl, das durch $C_1$–$C_5$-Alkylgruppen, Hydroxy oder Alkalisulfonatogruppen substituiert sein kann,
X Fluor, Chlor oder Brom,
m die Zahl 0 oder 1, aber nicht 0, wenn $R^1$ die Bedeutung Wasserstoff hat, und
n die Zahl 0 oder 1,
bedeuten, und, soweit ein Rest $R^4$ eine Aminogruppe enthält, deren Hydrohalogenide.

2. Arzneimittel enthaltend einen oder mehrere Metallkomplexe der allgemeinen Formel I′,

$$[R^{1\prime}(CH_2)_{m\prime}C(O)CR^{3\prime}C(O)R^{2\prime}]_2M'(OR^{4\prime})_{2-n}X'_n \quad (I'),$$

worin
M′ Titan, Zirkon oder Hafnium,
$R^{1\prime}$ Wasserstoff, $C_1$–$C_6$-Alkyl oder Phenyl, das durch Fluor, Chlor, Brom, Nitro, $C_1$–$C_2$-Alkyl, $C_1$–$C_2$-Alkoxy oder Trifluormethyl einfach oder mehrfach substituiert sein kann,
$R^{2\prime}$ $C_1$–$C_6$-Alkyl oder Phenyl, das durch $C_1$–$C_2$-Alkoxy substituiert sein kann,
$R^{3\prime}$ Wasserstoff oder Phenyl,
$R^{4\prime}$ $C_2$–$C_{16}$-Alkyl, das durch Hydroxy, $C_1$–$C_3$-Alkylamin oder Alkalisulfonatogruppen substituiert sein kann, oder Cyclohexyl, das durch $C_1$–$C_4$-Alkylgruppen substituiert sein kann,
X′ Fluor oder Chlor,
m′ die Zahl 0 oder 1, aber nicht 0, wenn $R^{1\prime}$ die Bedeutung Wasserstoff hat, und
n die Zahl 0 oder 1,
bedeuten, und, soweit ein Rest $R^{4\prime}$ eine Aminogruppe enthält, deren Hydrohalogenide.

3. Arzneimittel enthaltend einen oder mehrere Metallkomplexe der allgemeinen Formel I″,

$$[R^{1\prime\prime}(CH_2)_{m\prime\prime}C(O)CR^{3\prime\prime}C(O)R^{2\prime\prime}]_2M''(OR^{4\prime\prime})_{2-n}X''_n \quad (I''),$$

worin
M″ Titan, Zirkon oder Hafnium,
$R^{1\prime\prime}$ Wasserstoff, $C_1$–$C_4$-Alkyl oder Phenyl, das durch $C_1$–$C_2$-Alkoxy substituiert sein kann,
$R^{2\prime\prime}$ $C_1$–$C_4$-Alkyl oder Phenyl,
$R^{3\prime\prime}$ Wasserstoff oder Phenyl,
$R^{4\prime\prime}$ $C_2$–$C_{13}$-Alkyl, das durch ein Hydroxy, ein Di-$C_1$–$C_3$-alkylamin oder eine Alkalisulfonatogruppe substituiert sein kann, oder 1-Isopropyl-4-methyl-cyclohexyl-2,
X″ Chlor,
m″ die Zahl 1, falls $R^{1\prime\prime}$ Wasserstoff bedeutet, und die Zahl 0, falls $R^{1\prime\prime}$ Phenyl oder $C_1$–$C_4$-Alkyl bedeutet,
n die Zahl 0 oder 1 bedeuten und, soweit ein Rest $R^{4\prime\prime}$ einen Aminorest enthält, deren Hydrochloride.

4. Arzneimittel enthaltend Diethoxybis-(1-phenyl-1,3-butandionato)-titan (IV).

5. Verbindungen der allgemeinen Formel $I^a$,

$$[R^{1a}(CH_2)_{m^a}C(O)CR^{3a}C(O)R^{2a}]_2M^a(OR^{4a})_{2-n^a}X^a_{n^a} \quad (I^a),$$

worin
$M^a$ Titan, Zirkon oder Hafnium,
$R^{1a}$ Wasserstoff, $C_1$–$C_8$-Alkyl oder Phenyl, das durch Fluor, Chlor, Brom, Nitro, $C_1$–$C_4$-Alkyl, $C_1$–$C_4$-Alkoxy oder Trifluormethyl einfach oder mehrfach substituiert sein kann,
$R^{2a}$ $C_1$–$C_8$-Alkyl oder Phenyl, das durch Fluor, Chlor, Brom, Nitro, $C_1$–$C_4$-Alkyl, $C_1$–$C_4$-Alkoxy oder Trifluormethyl einfach oder mehrfach sub-

stituiert sein kann,
$R^{3a}$ Wasserstoff oder Phenyl,
$R^{4a}$ $C_1$–$C_{18}$-Alkyl, das durch Hydroxy, $C_1$–$C_3$-Alkylamin oder Alkalisulfonatogruppen substituiert sein kann, oder $C_5$–$C_8$-Cycloalkyl, das durch $C_1$–$C_5$-Alkylgruppen, Hydroxy oder Alkalisulfonatogruppen substituiert sein kann, aber nicht unsubstituiertes $C_1$–$C_5$- oder $C_{18}$-Alkyl, falls $M^a$ Titan, $R^{3a}$ Wasserstoff, $m^a$ und $n^a$ die Zahl 0 und $R^{1a}$ $C_1$- oder $C_4$-Alkyl oder Phenyl und $R^{2a}$ $C_1$–$C_6$-Alkyl oder Phenyl bedeuten, und nicht unsubstituiertes $C_3$-Alkyl, falls $M^a$ Zirkon, $R^{3a}$ Wasserstoff, $m^a$ und $n^a$ die Zahl 0 und $R^{1a}$ und $R^{2a}$ unabhängig voneinander Methyl oder Phenyl bedeuten,
$X^a$ Fluor, Chlor oder Brom,
$m^a$ die Zahl 0 oder 1, aber nicht 0, wenn $R^{1a}$ die Bedeutung Wasserstoff hat, und
$n^a$ die Zahl 0 oder 1,
bedeuten und, soweit $R^{4a}$ eine Aminogruppe enthält, deren Hydrohalogenide.

6. Verbindungen der allgemeinen Formel $I^{a'}$,

$$[R^{1a'}(CH_2)_{m^{a'}}C(O)CR^{3a'}C(O)R^{2a'}]_2M^{a'}(OR^{4a'})_{2-n^{a'}}X^{a'}_{n^{a'}} \quad (I^{a'}),$$

worin
$M^{a'}$ Titan, Zirkon oder Hafnium,
$R^{1a'}$ Wasserstoff, $C_1$–$C_4$-Alkyl oder Phenyl, das durch $C_1$–$C_2$-Alkoxy substituiert sein kann,
$R^{2a'}$ Methyl oder Phenyl,
$R^{3a'}$ Wasserstoff oder Phenyl,
$R^{4a'}$ $C_2$–$C_{16}$-Alkyl, das durch Hydroxy, Di-$C_1$–$C_3$-alkylamin oder eine Alkalisulfonatogruppe substituiert sein kann, oder 1-Isopropyl-4-methyl-cyclohexyl-2, aber nicht unsubstituiertes $C_2$–$C_5$-Alkyl, falls $M^{a'}$ Titan, $R^{3a'}$ Wasserstoff, $m^{a'}$ und $n^{a'}$ die Zahl 0 und $R^{1a'}$ $C_1$- oder $C_4$-Alkyl oder Phenyl bedeuten, und nicht unsubstituiertes $C_3$-Alkyl, falls $M^{a'}$ Zirkon, $R^{3a'}$ Wasserstoff, $m^{a'}$ und $n^{a'}$ die Zahl 0 und $R^{1a'}$ Methyl oder Phenyl bedeuten,
$X^{a'}$ Chlor,
$m^{a'}$ die Zahl 0 oder 1, aber nicht 0, wenn $R^{1a'}$ die Bedeutung Wasserstoff hat, und
$n^{a'}$ die Zahl 0 oder 1,
bedeuten und, soweit $R^{4a'}$ eine Aminogruppe enthält, deren Hydrochloride.

7. Chloro-(2,3-dimethyl-2,3-butandiolato)-bis-(1-phenyl-1,3-butandionato)-titan(IV).

8. Chloro-(2-hydroxypropanolato)-bis-(1-phenyl-1,3-butandionato)-titan(IV).

9. Diethoxy-bis-(3-phenyl-2,4-pentandionato)-titan(IV).

10. Bis-(tridecanolato)-bis-(1-phenyl-1,3-butandionato)-titan(IV).

11. Verfahren zur Herstellung der Verbindungen nach Anspruch 5 oder 6, das dadurch gekennzeichnet ist, dass man

a) eine Tetraalkoxymetall(IV)verbindung $M^a(OR^{4a})_4$ bzw. $M^{a'}(OR^{4a'})_4$, wobei $M^a$ bzw. $M^{a'}$, $R^{4a}$ bzw. $R^{4a'}$ die vorstehend angegebenen Bedeutungen haben, unter Feuchtigkeitsausschluss in einem inerten Lösungsmittel mit einem Diketon $R^{1a}(CH_2)_{m^a}C(O)CHR^{3a}C(O)R^{2a}$ bzw. $R^{1a'}(CH_2)_{m^{a'}}C(O)CHR^{3a'}C(O)R^{2a'}$, wobei $R^{1a}$ bzw. $R^{1a'}$, $R^{2a}$ bzw. $R^{2a'}$, $R^{3a}$ bzw. $R^{3a'}$ und $m^a$ bzw. $m^{a'}$ die vorstehend angegebenen Bedeutungen haben,

b) eine Dihalogenobis-(diketonato)-metall(IV)-verbindung $[R^{1a}(CH_2)_{m^a}C(O)CR^{3a}C(O)R^{2a}]_2M^aX^a$ bzw. $[R^{1a'}(CH_2)_{m^{a'}}C(O)CR^{3a'}C(O)R^{2a'}]_2M^{a'}X^{a'}_2$, worin $M^a$ bzw. $M^{a'}$, $R^{1a}$ bzw. $R^{1a'}$, $R^{2a}$ bzw. $R^{2a'}$, $R^{3a}$ bzw. $R^{3a'}$, $X^a$ bzw. $X^{a'}$ und $m^a$ bzw. $m^{a'}$ die vorstehend angegebenen Bedeutungen haben, mit einem Alkohol $HOR^{4a}$ bzw. $HOR^{4a'}$ oder dem entsprechenden Alkalialkoholat, worin $R^{4a}$ bzw. $R^{4a'}$ die oben angegebenen Bedeutungen haben, umsetzt, oder

c) eine Verbindung der allgemeinen Formel $I^a$ bzw. $I^{a'}$, worin $n^a$ bzw. $n^{a'}$ 0 bedeuten und $M^a$ bzw. $M^{a'}$, $R^{1a}$ bzw. $R^{1a'}$, $R^{2a}$ bzw. $R^{2a'}$, $R^{3a}$ bzw. $R^{3a'}$, $R^{4a}$ bzw. $R^{4a'}$ und $m^a$ bzw. $m^{a'}$ die vorstehend angegebenen Bedeutungen haben, mit einem Alkohol $HOR^{4a}$ bzw. $HOR^{4a'}$, worin $R^{4a}$ bzw. $R^{4a'}$ die oben angegebenen Bedeutungen hat, umsetzt.

12. Arzneimittel enthaltend eine oder mehrere Komplexverbindungen nach einem oder mehreren der Ansprüche 7 bis 10.

13. Kopräzipitate erhalten aus den Metallkomplexen der allgemeinen Formel I,

$$[R^1(CH_2)_mC(O)CR^3C(O)R^2]_2M(OR^4)_{2-n}X_n \quad (I),$$

worin
M Titan, Zirkon oder Hafnium,
$R^1$ Wasserstoff, $C_1$–$C_8$-Alkyl oder Phenyl, das durch Fluor, Chlor, Brom, Nitro, $C_1$–$C_4$-Alkyl, $C_1$–$C_4$-Alkoxy oder Trifluormethyl einfach oder mehrfach substituiert sein kann,
$R^2$ $C_1$–$C_8$-Alkyl oder Phenyl, das durch Fluor, Chlor, Brom, Nitro, $C_1$–$C_4$-Alkyl, $C_1$–$C_4$-Alkoxy oder Trifluormethyl einfach oder mehrfach substituiert sein kann,
$R^3$ Wasserstoff oder Phenyl,
$R^4$ $C_1$–$C_{18}$-Alkyl, das durch Hydroxy, $C_1$–$C_3$-Alkylamin oder Alkalisulfonatogruppen substituiert sein kann, oder $C_5$–$C_8$-Cycloalkyl, das durch $C_1$–$C_5$-Alkylgruppen, Hydroxy oder Alkalisulfonatogruppen substituiert sein kann,
X Fluor, Chlor oder Brom,
m die Zahl 0 oder 1, aber nicht 0, wenn $R^1$ die Bedeutung Wasserstoff hat, und
n die Zahl 0 oder 1,
bedeuten und, soweit ein Rest $R^4$ eine Aminogruppe enthält, deren Hydrohalogeniden und hydrophilen Polymeren.

14. Kopräzipitate nach Anspruch 13, dadurch gekennzeichnet, dass es sich bei den hydrophilen Polymeren um Polyvinylpyrrolidon handelt.

15. Kopräzipitate nach Anspruch 13, dadurch gekennzeichnet, dass es sich bei den hydrophilen Polymeren um Polyoxyethylensorbitanmonofettsäureester (Tween®) handelt.

16. Kopräzipitate nach Anspruch 13, dadurch

gekennzeichnet, dass es sich bei den hydrophilen Polymeren um Glycerin-Polyethylenglykolricinoleat handelt.

17. Kopräzipitat aus Diethoxybis-(1-phenyl-1,3-butandionato)-titan(IV) und Glycerin-Polyethylenglykolricinoleat (Cremophor®EL).

18. Verfahren zur Herstellung von Kopräzipitaten nach einem der Ansprüche 13 bis 17, dadurch gekennzeichnet, dass man eine Lösung eines Metallkomplexes der allgemeinen Formel I,

$$[R^1(CH_2)_mC(O)CR^3C(O)R^2]_2M(OR^4)_{2-n}X_n \quad (I),$$

worin
M Titan, Zirkon oder Hafnium,
$R^1$ Wasserstoff, $C_1$–$C_8$-Alkyl oder Phenyl, das durch Fluor, Chlor, Brom, Nitro, $C_1$–$C_4$-Alkyl, $C_1$–$C_4$-Alkoxy oder Trifluormethyl einfach oder mehrfach substituiert sein kann,
$R^2$ $C_1$–$C_8$-Alkyl oder Phenyl, das durch Fluor, Chlor, Brom, Nitro, $C_1$–$C_4$-Alkyl, $C_1$–$C_4$-Alkoxy oder Trifluormethyl einfach oder mehrfach substituiert sein kann.
$R^3$ Wasserstoff oder Phenyl,
$R^4$ $C_1$–$C_{18}$-Alkyl, das durch Hydroxy, $C_1$–$C_3$-Alkylamin oder Alkalisulfonatogruppen substituiert sein kann, oder $C_5$–$C_8$-Cycloalkyl, das durch $C_1$–$C_5$-Alkylgruppen, Hydroxy oder Alkalisulfonatogruppen substituiert sein kann,
X Fluor, Chlor oder Brom,
m die Zahl 0 oder 1, aber nicht 0, wenn $R^1$ die Bedeutung Wasserstoff hat, und
n die Zahl 0 oder 1,
bedeuten und, soweit ein Rest $R^4$ eine Aminogruppe enthält, deren Hydrohalogenide und eines hydrophilen Polymeren in einem inerten organischen Lösungsmittel bis zur Trockne einengt.

19. Verfahren nach Anspruch 18, dadurch gekennzeichnet, dass man zur Lösung des Metallkomplexes und des hydrophilen Polymeren vor dem Einengen Propylenglykol oder Butylenglykol zugibt.

20. Sterile wässrige Lösungen enthaltend Kopräzipitate nach einem der Ansprüche 13 bis 17.

21. Verwendung der in den Ansprüchen 1 bis 10 angegebenen Metallkomplexe zur Herstellung von Arzneimitteln zur Behandlung von Krebskrankheiten.

22. Verwendung der in den Ansprüchen 13 bis 17 angegebenen Kopräzipitate zur Herstellung von Arzneimitteln zur Behandlung von Krebskrankheiten.

**Patentansprüche für den Vertragsstaat AT**

1. Verfahren zur Herstellung von Arzneimitteln zur Behandlung von Krebskrankheiten, dadurch gekennzeichnet, dass man Metallkomplexe der allgemeinen Formel I

$$[R^1(CH_2)_mC(O)CR^3C(O)R^2]_2M(OR^4)_{2-n}X_n \quad (I),$$

worin
M Titan, Zirkon oder Hafnium,
$R^1$ Wasserstoff, $C_1$–$C_8$-Alkyl oder Phenyl, das durch Fluor, Chlor, Brom, Nitro, $C_1$–$C_4$-Alkyl, $C_1$–$C_4$-Alkoxy oder Trifluormethyl einfach oder mehrfach substituiert sein kann,
$R^2$ $C_1$–$C_8$-Alkyl oder Phenyl, das durch Fluor, Chlor, Brom, Nitro, $C_1$–$C_4$-Alkyl, $C_1$–$C_4$-Alkoxy oder Trifluormethyl einfach oder mehrfach substituiert sein kann,
$R^3$ Wasserstoff oder Phenyl,
$R^4$ $C_1$–$C_{18}$-Alkyl, das durch Hydroxy, $C_1$–$C_3$-Alkylamin oder Alkalisulfonatogruppen substituiert sein kann, oder $C_5$–$C_8$-Cycloalkyl, das durch $C_1$–$C_5$-Alkylgruppen, Hydroxy oder Alkalisulfonatogruppen substituiert sein kann,
X Fluor, Chlor oder Brom,
m die Zahl 0 oder 1, aber nicht 0, wenn $R^1$ die Bedeutung Wasserstoff hat, und
n die Zahl 0 oder 1,
bedeuten oder, soweit ein Rest $R^4$ eine Aminogruppe enthält, deren Hydrohalogenide herstellt, indem man

a) eine Tetralkoxymetall(IV)verbindung $M(OR^4)_4$, wobei M und $R^4$ die vorstehend angegebenen Bedeutungen haben, unter Feuchtigkeitsausschluss in einem inerten Lösungsmittel mit einem Diketon $R^1(CH_2)_mC(O)CHR^3C(O)R^2$, wobei $R^1$, $R^2$, $R^3$ und m die vorstehend angegebenen Bedeutungen haben, umsetzt,
b) eine Dihalogenobis-(diketonato)-metall-(IV)-verbindung $[R^1(CH_2)_mC(O)CR^3C(O)R^2]_2MX_2$, worin M, $R^1$, $R^2$, $R^3$, X und m die vorstehend angegebenen Bedeutungen haben, mit einem Alkohol $HOR^4$ oder dem entsprechenden Alkalialkoholat, worin $R^4$ die oben angegebene Bedeutung hat, umsetzt, oder
c) eine Verbindung der allgemeinen Formel I, worin n 0 bedeutet und M, $R^1$, $R^2$, $R^3$, $R^4$ und m die vorstehend angegebenen Bedeutungen haben, mit einem Alkohol $HOR^4$, worin $R^4$ die oben angegebenen Bedeutungen hat, umsetzt,

und dass einer oder mehrere der so erhaltenen Metallkomplexe der Formel I durch Mischen mit einem geeigneten pharmazeutisch annehmbaren Trägerstoff in eine zur Verabreichung als Arzneimittel zur Behandlung von Krebskrankheiten geeignete Form gebracht werden.

2. Verfahren zur Herstellung von Arzneimitteln zur Behandlung von Krebskrankheiten, dadurch gekennzeichnet, dass man Metallkomplexe der allgemeinen Formel I′

$$[R^{1'}(CH_2)_{m'}C(O)CR^{3'}C(O)R^{2'}]_2M'(OR^{4'})_{2-n}X'_n \quad (I'),$$

worin
M′ Titan, Zirkon oder Hafnium,
$R^{1'}$ Wasserstoff, $C_1$–$C_6$-Alkyl oder Phenyl, das durch Fluor, Chlor, Brom, Nitro, $C_1$–$C_2$-Alkyl, $C_1$–$C_2$-Alkoxy oder Trifluormethyl einfach oder mehrfach substituiert sein kann,
$R^{2'}$ $C_1$–$C_6$-Alkyl oder Phenyl, das durch $C_1$–$C_2$-Alkoxy substituiert sein kann,
$R^{3'}$ Wasserstoff oder Phenyl,
$R^{4'}$ $C_2$–$C_{16}$-Alkyl, das durch Hydroxy, $C_1$–$C_3$-Alkyl-

amin oder Alkalisulfonatogruppen substituiert sein kann, oder Cyclohexyl, das durch $C_1$–$C_4$-Alkylgruppen substituiert sein kann,
X′ Fluor oder Chlor,
m′ die Zahl 0 oder 1, aber nicht 0, wenn $R^{1'}$ die Bedeutung Wasserstoff hat, und
n die Zahl 0 oder 1,
bedeuten oder, soweit ein Rest $R^{4'}$ eine Aminogruppe enthält, deren Hydrohalogenide, herstellt, indem man

a) eine Tetralkoxymetall(IV)verbindung $M'(OR^{4'})_4$, wobei M′ und $R^{4'}$ die vorstehend angegebenen Bedeutungen haben, unter Feuchtigkeitsausschluss in einem inerten Lösungsmittel mit einem Diketon $R^{1'}(CH_2)_{m'}C(O)CHR^{3'}C(O)R^{2'}$, wobei $R^{1'}$, $R^{2'}$, $R^{3'}$ und m′ die vorstehend angegebenen Bedeutungen haben, umsetzt,
b) eine Dihalogenobis-(diketonato)-metall-(IV)-verbindung $[R^{1'}(CH_2)_{m'}C(O)CR^{3'}C(O)R^{2'}]_2M'X'_2$, worin M′, $R^{1'}$, $R^{2'}$, $R^{3'}$, X′ und m′ die vorstehend angegebenen Bedeutungen haben, mit einem Alkohol $HOR^{4'}$ oder dem entsprechenden Alkalialkoholat, worin $R^{4'}$ die oben angegebene Bedeutung hat, umsetzt, oder
c) eine Verbindung der allgemeinen Formel I′, worin n′ 0 bedeutet und M′, $R^{1'}$, $R^{2'}$, $R^{3'}$, $R^{4'}$ und m′ die vorstehend angegebenen Bedeutungen haben, mit einem Alkohol $HOR^{4'}$, worin $R^{4'}$ die oben angegebenen Bedeutungen hat, umsetzt,

und dass einer oder mehrere der so erhaltenen Metallkomplexe der Formel I′ durch Mischen mit einem geeigneten pharmazeutisch annehmbaren Trägerstoff in eine zur Verabreichung als Arzneimittel zur Behandlung von Krebskrankheiten geeignete Form gebracht werden.

3. Verfahren zur Herstellung von Arzneimitteln zur Behandlung von Krebskrankheiten, dadurch gekennzeichnet, dass man Metallkomplexe der allgemeinen Formel I″

$$[R^{1''}(CH_2)_{m''}C(O)CR^{3''}C(O)R^{2''}]_2M''(OR^{4''})_{2-n}X''_n \quad (I''),$$

worin
M″ Titan, Zirkon oder Hafnium,
$R^{1''}$ Wasserstoff, $C_1$–$C_4$-Alkyl oder Phenyl, das durch $C_1$–$C_2$-Alkoxy substituiert sein kann,
$R^{2''}$ $C_1$–$C_4$-Alkyl oder Phenyl,
$R_{3''}$ Wasserstoff oder Phenyl,
$R^{4''}$ $C_2$–$C_{13}$-Alkyl, das durch ein Hydroxy, ein Di-$C_1$–$C_3$-alkylamin oder eine Alkalisulfonatogruppe substituiert sein kann, oder 1-Isopropyl- 4-methyl-cyclohexyl-2,
X″ Chlor,
m″ die Zahl 0 oder 1, aber nicht 0, wenn $R^{1''}$ die Bedeutung Wasserstoff hat,
n die Zahl 0 oder 1,
bedeuten, oder, soweit ein Rest $R^{4''}$ einen Aminorest enthält, deren Hydrohalogenide herstellt, indem man

a) eine Tetralkoxymetall(IV)verbindung $M''(OR^{4''})_4$, wobei M″ und $R^{4''}$ die vorstehend angegebenen Bedeutungen haben, unter Feuchtigkeitsausschluss in einem inerten Lösungsmittel mit einem Diketon $R^{1''}(CH_2)_{m''}C(O)CHR^{3''}C(O)R^{2''}$, wobei $R^{1''}$, $R^{2''}$, $R^{3''}$ und m″ die vorstehend angegebenen Bedeutungen haben, umsetzt.
b) eine Dihalogenobis-(diketonato)-metall-(IV)-verbindung
$[R^{1''}(CH_2)_{m''}C(O)CR^{3''}C(O)R^{2''}]_2M''X''_2$,
worin M″, $R^{1''}$, $R^{2''}$, $R^{3''}$, X″ und m″ die vorstehend angegebenen Bedeutungen haben, mit einem Alkohol $HOR^{4''}$ oder dem entsprechenden Alkalialkoholat, worin $R^{4''}$ die oben angegebene Bedeutung hat, umsetzt, oder
c) eine Verbindung der allgemeinen Formel I″, worin n″ 0 bedeutet und M″, $R^{1''}$, $R^{2''}$, $R^{3''}$, $R^{4''}$ und m″ die vorstehend angegebenen Bedeutungen haben, mit einem Alkohol $HOR^{4''}$, worin $R^{4''}$ die oben angegebenen Bedeutungen hat, umsetzt,

und dass einer oder mehrere der so erhaltenen Metallkomplexe der Formel I″ durch Mischen mit einem geeigneten pharmazeutisch annehmbaren Trägerstoff in eine zur Verabreichung als Arzneimittel zur Behandlung von Krebskrankheiten geeignete Form gebracht werden.

4. Verfahren zur Herstellung von antineoplastisch wirksamen Mitteln, dadurch gekennzeichnet, dass man die Komplexverbindung Diethoxybis(benzoylacetonato)titan(IV) herstellt, indem man Titan(IV)tetraethoxid unter Feuchtigkeitsausschluss in einem inerten Lösungsmittel mit Benzoylaceton im molaren Verhältnis von 1:2 umsetzt und dass Diethoxybis(benzoylacetonato)titan(IV) durch Mischen mit einem geeigneten pharmazeutisch annehmbaren Trägerstoff in eine zur Verabreichung als Arzneimittel zur Behandlung von Krebskrankheiten geeignete Form gebracht wird.

5. Verfahren zur Herstellung der Verbindungen der allgemeinen Formel $I^a$

$$[R^{1a}(CH_2)_{m^a}C(O)CR^{3a}C(O)R^{2a}]_2M^a)(OR^{4a})_{2-n^a}X^a_{n^a} \quad (Ia),$$

$M^a$ Titan, Zirkon oder Hafnium,
$R^{1a}$ Wasserstoff, $C_1$–$C_8$-Alkyl oder Phenyl, das durch Fluor, Chlor, Brom, Nitro, $C_1$–$C_4$-Alkyl oder Trifluormethyl einfach oder mehrfach substituiert sein kann,
$R^{2a}$ $C_1$–$C_8$-Alkyl oder Phenyl, das durch Fluor, Chlor, Brom, Nitro, $C_1$–$C_4$-Alkyl, $C_1$–$C_4$-Alkoxy oder Trifluormethyl einfach oder mehrfach substituiert sein kann,
$R^{3a}$ Wasserstoff oder Phenyl,
$R^{4a}$ $C_1$–$C_{18}$-Alkyl, das durch Hydroxy, $C_1$–$C_3$-Alkylamin oder Alkalisulfonatogruppen substituiert sein kann, oder $C_5$–$C_8$-Cycloalkyl, das durch $C_1$–$C_5$-Alkylgruppen, Hydroxy oder Alkalisulfonatogruppen substituiert sein kann, aber nicht unsubstituiertes $C_1$–$C_5$- oder $C_{18}$-Alkyl, falls $M^a$ Titan, $R^{3a}$ Wasserstoff, $m^a$ und $n^a$ die Zahl 0 und $R^{1a}$ $C_1$- oder $C_4$-Alkyl oder Phenyl und $R^{2a}$ $C_1$–$C_6$-Alkyl oder Phenyl bedeuten, und nicht unsubstituiertes $C_3$-Alkyl, falls $M^a$ Zirkon, $R^{3a}$ Wasserstoff, $m^a$ und $n^a$ die Zahl 0 und $R^{1a}$ und $R^{2a}$ unabhängig voneinander Methyl oder Phenyl bedeuten,
$X^a$ Fluor, Chlor oder Brom,
$m^a$ die Zahl 0 oder 1, aber nicht 0, wenn $R^{1a}$ die Bedeutung Wasserstoff hat, und
n die Zahl 0 oder 1,
bedeuten, oder, soweit $R^{4a}$ eine Aminogruppe enthält, deren Hydrohalogenide, dadurch gekennzeichnet, dass man

a) eine Tetralkoxymetall(IV)verbindung $M^a(OR^{4a})_4$, wobei $M^a$ und $R^{4a}$ die vorstehend angegebenen Bedeutungen haben, unter Feuchtigkeitsausschluss in einem inerten Lösungsmittel mit einem Diketon
$R^{1a}(CH_2)_{m^a}C(O)CHR^{3a}C(O)R^{2a}$,
wobei $R^{1a}$, $R^{2a}$, $R^{3a}$ und $m^a$ die vorstehend angegebenen Bedeutungen haben, umsetzt,
b) eine Dihalogenobis-(diketonato)-metall(IV)-verbindung
$[R^{1a}(CH_2)_{m^a}\text{-}C(O)CR^{3a}C(O)R^{2a}]_2M^aX^a{}_2$,
worin $M^a$, $R^{1a}$, $R^{2a}$, $R^{3a}$, $X^a$ und $m^a$ die vorstehend angegebenen Bedeutungen haben, mit einem Alkohol $HOR^{4a}$ oder dem entsprechenden Alkalialkoholat, worin $R^{4a}$ die oben angegebene Bedeutung hat, umsetzt, oder
c) eine Verbindung der allgemeinen Formel $I^a$, worin $n^a$ 0 bedeutet und $M^a$, $R^{1a}$, $R^{2a}$, $R^{3a}$, und $m^a$ die vorstehend angegebenen Bedeutungen haben, mit einem Alkohol $HOR^{4a}$ worin $R^{4a}$ die oben angegebenen Bedeutungen hat, umsetzt.

6. Verfahren zur Herstellung der Verbindungen der allgemeinen Formel $I^{a'}$,

$$[R^{1a'}(CH_2)_{m^{a'}}C(O)CR^{3a'}C(O)R^{2a'}]_2M^{a'}(OR^{4a'})_{2-n^{a'}}X^{a'}{}_{n^{a'}} \qquad (I^{a'}),$$

worin
$M^{a'}$ Titan, Zirkon oder Hafnium,
$R^{1a'}$ Wasserstoff, $C_1$–$C_4$-Alkyl oder Phenyl, das durch $C_1$–$C_2$-Alkoxy substituiert sein kann,
$R^{2a'}$ Methyl oder Phenyl,
$R^{3a'}$ Wasserstoff oder Phenyl,
$R^{4a'}$ $C_2$–$C_{16}$-Alkyl, das durch Hydroxy, Di-$C_1$–$C_3$-alkylamin oder Alkalisulfonatogruppen substituiert sein kann, oder 1-Isopropyl-4-methyl-cyclohexyl-2, aber nicht unsubstituiertes $C_2$–$C_5$-Alkyl, falls $M^{a'}$ Titan, $R^{3a'}$ Wasserstoff, $m^{a'}$ und $n^{a'}$ die Zahl 0 und $R^{1a'}$ $C_1$- oder $C_4$-Alkyl oder Phenyl bedeuten, und nicht unsubstituiertes $C_3$-Alkyl, falls $M^{a'}$ Zirkon, $R^{3a'}$ Wasserstoff, $m^{a'}$ und $n^{a'}$ die Zahl 0 und $R^{1a'}$ Methyl oder Phenyl bedeuten,
$X^{a'}$ Chlor,
$m^{a'}$ die Zahl 0 oder 1, aber nicht 0, wenn $R^{1a'}$ die Bedeutung Wasserstoff hat, und
$n^{a'}$ die Zahl 0 oder 1,
bedeuten, oder, soweit ein Rest $R^{4a'}$ eine Aminogruppe enthält, deren Hydrochloride, dadurch gekennzeichnet, dass man

a) eine Tetralkoxymetall(IV)verbindung $M^{a'}(OR^{4a'})_4$, wobei $M^{a'}$ und $R^{4a'}$ die vorstehend angegebenen Bedeutungen haben, unter Feuchtigkeitsausschluss in einem inerten Lösungsmittel mit einem Diketon
$R^{1a'}(CH_2)_{m^{a'}}C(O)CHR^{3a'}C(O)R^{2a'}$,
wobei $R^{1a'}$, $R^{2a'}$, $R^{3a'}$ und $m^{a'}$ die vorstehend angegebenen Bedeutungen haben, umsetzt,
b) eine Dihalogenobis-(diketonato)-metall(IV)-verbindung
$[R^{1a'}(CH_2)_{m^{a'}}3C(O)CR^{3a'}C(O)R^{2a'}]_2M^{a'}X^{a'}{}_2$,
worin $M^{a'}$, $R^{1a'}$, $R^{2a'}$, $R^{3a'}$, $X^{a'}$ und $m^{a'}$ die vorstehend angegebenen Bedeutungen haben, mit einem Alkohol $HOR^{4a'}$ oder dem entsprechenden Alkalialkoholat, worin $R^{4a'}$ die oben angegebene Bedeutung hat, umsetzt, oder
c) eine Verbindung der allgemeinen Formel $I^{a'}$, worin $n^{a'}$ 0 bedeutet und $M^{a'}$, $R^{1a'}$, $R^{2a'}$, $R^{3a'}$ und $m^{a'}$ die vorstehend angegebenen Bedeutungen haben, mit einem Alkohol $HOR^{4a'}$, worin $R^{4a'}$ die oben angegebenen Bedeutungen hat, umsetzt.

7. Verfahren zur Herstellung von Kopräzipitaten der Metallkomplexe der allgemeinen Formel I,

$$[R^1(CH_2)_mC(O)CR^3C(O)R^2]_2M(OR^4)_{2-n}X_n \qquad (I),$$

worin
M Titan, Zirkon oder Hafnium,
$R^1$ Wasserstoff, $C_1$–$C_8$-Alkyl oder Phenyl, das durch Fluor, Chlor, Brom, Nitro, $C_1$–$C_4$-Alkyl, $C_1$–$C_4$-Alkoxy oder Trifluormethyl einfach oder mehrfach substituiert sein kann,
$R^2$ $C_1$–$C_8$-Alkyl oder Phenyl, das durch Fluor, Chlor, Brom, Nitro, $C_1$–$C_4$-Alkyl, $C_1$–$C_4$-Alkoxy oder Trifluormethyl einfach oder mehrfach substituiert sein kann,
$R^3$ Wasserstoff oder Phenyl,
$R^4$ $C_1$–$C_{18}$-Alkyl, das durch Hydroxy, $C_1$–$C_3$-Alkylamin oder Alkalisulfonatogruppen substituiert sein kann, oder $C_5$–$C_8$-Cycloalkyl, das durch $C_1$–$C_5$-Alkylgruppen, Hydroxy oder Alkalisulfonatogruppen substituiert sein kann,
X Fluor, Chlor oder Brom,
m die Zahl 0 oder 1, aber nicht 0, wenn $R^1$ die Bedeutung Wasserstoff hat, und
n die Zahl 0 oder 1,
bedeuten oder, soweit ein Rest $R^4$ eine Aminogruppe enthält, deren Hydrohalogeniden mit hydrophilen Polymeren, dadurch gekennzeichnet, dass man eine Lösung des Metallkomplexes und eines hydrophilen Polymeren in einem inerten organischen Lösungsmittel bis zur Trockne einengt.

8. Verfahren nach Patentanspruch 7, dadurch gekennzeichnet, dass als hydrophiles Polymer Polyvinylpyrolidon eingesetzt wird.

9. Verfahren nach Patentanspruch 7, dadurch gekennzeichnet, dass als hydrophiles Polymer Polyoxyethylensorbitanmonofettsäureester eingesetzt wird.

10. Verfahren nach Patentanspruch 7, dadurch gekennzeichnet, dass als hydrophiles Polymer Glycerin-Polyethylenglykolricinoleat eingesetzt wird.

11. Verfahren nach einem der Patentansprüche 7 bis 10, dadurch gekennzeichnet, dass man zur Lösung des Metallkomplexes und des hydrophilen Polymeren vor dem Einengen Propylenglykol oder Butylenglykol zugibt.

12. Verfahren nach einem der Patentansprüche 7 bis 11, dadurch gekennzeichnet, dass als Metallkomplex Diethoxybis(benzoylacetonato)titan(IV) eingesetzt wird.

13. Verfahren nach einem der Patentansprüche 7 bis 12, dadurch gekennzeichnet, dass als inertes Lösungsmittel Ethanol eingesetzt wird.

14. Verwendung der Metallkomplexe der in den Ansprüchen 1, 2, 3, 5 bzw. 6 angegebenen allgemeinen Formeln I, I′, I″, $I^a$ und $I^{a'}$ zur Herstellung von Arzneimitteln zur Behandlung von Krebskrankheiten.

15. Verwendung von Diethoxybis-(1-phenyl-1,3-butandionato)titan(IV) zur Herstellung von Arzneimitteln zur Behandlung von Krebskrankheiten.

16. Verwendung von Kopräzipitaten der Metallkomplexe der in Anspruch 7 angegebenen allgemeinen Formel I mit hydrophilen Polymeren zur Herstellung von Arzneimitteln zur Behandlung von Krebskrankheiten.

**Claims for the contracting states BE, CH, DE, FR, GB, IT, LI, LU, NL, SE**

1. Medicaments containing one or more metal complexes of the general formula I

$$[R^1(CH_2)_mC(O)CR^3C(O)R^2]_2M(OR^4)_{2-n}X_n \quad (I)$$

wherein
M denotes titanium, zirconium or hafnium,
$R^1$ denotes hydrogen, $C_1$–$C_8$-alkyl or phenyl, which can be monosubstituted or polysubstituted by fluorine, chlorine, bromine, nitro, $C_1$–$C_4$-alkyl, $C_1$–$C_4$-alkoxy or trifluoromethyl,
$R^2$ denotes $C_1$–$C_8$-alkyl, or phenyl, which can be monosubstituted or polysubstituted by fluorine, chlorine, bromine, nitro, $C_1$–$C_4$-alkyl, $C_1$–$C_4$-alkoxy or trifluoromethyl,
$R^3$ denotes hydrogen or phenyl,
$R^4$ denotes $C_1$–$C_{18}$-alkyl, which can be substituted by hydroxyl, $C_1$–$C_3$-alkylamino or alkali metal sulfonato groups, or denotes $C_5$–$C_8$-cycloalkyl, which can be substituted by $C_1$–$C_5$-alkyl groups, hydroxyl or alkali metal sulfonato groups,
X denotes fluorine, chlorine or bromine,
m denotes the number 0 or 1, but does not denote 0 if $R^1$ denotes hydrogen, and
n denotes the number 0 or 1,
and, if a radical $R^4$ contains an amino group, hydrohalides thereof.

2. Medicaments containing one or more metal complexes of the general formula I′

$$[R^{1'}(CH_2)_{m'}C(O)CR^{3'}C(O)R^{2'}]_2M'(OR^{4'})_{2-n}X'_n \quad (I')$$

wherein
M′ denotes titanium, zirconium or hafnium,
$R^{1'}$ denotes hydrogen, $C_1$–$C_6$-alkyl or phenyl, which can be monosubstituted or polysubstituted by fluorine, chlorine, bromine, nitro, $C_1$–$C_2$-alkyl, $C_1$–$C_2$-alkoxy or trifluoromethyl,
$R^{2'}$ denotes $C_1$–$C_6$-alkyl or phenyl, which can be substituted by $C_1$–$C_2$-alkoxy,
$R^{3'}$ denotes hydrogen or phenyl,
$R^{4'}$ denotes $C_2$–$C_{16}$-alkyl, which can be substituted by hydroxyl, $C_1$–$C_3$-alkylamino or alkali metal sulfonato groups, or denotes cyclohexyl, which can be substituted by $C_1$–$C_4$-alkyl groups,
X′ denotes fluorine or chlorine,
m′ denotes the number 0 or 1, but does not denote 0 if $R^{1'}$ denotes hydrogen, and
n denotes the number 0 or 1,
and, if a radical $R^{4'}$ contains an amino group, hydrohalides thereof.

3. Medicaments containing one or more metal complexes of the general formula I″

$$[R^{1''}(CH_2)_{m''}C(O)CR^{3''}C(O)R^{2''}]_2M''(OR^{4''}2)_{2-n}X''_n \quad (I'')$$

wherein
M″ denotes titanium, zirconium or hafnium,
$R^{1''}$ denotes hydrogen, $C_1$–$C_4$-alkyl or phenyl, which can be substituted by $C_1$–$C_2$-alkoxy,
$R^{2''}$ denotes $C_1$–$C_4$-alkyl or phenyl,
$R^{3''}$ denotes hydrogen or phenyl,
$R^{4''}$ denotes $C_2$–$C_{13}$-alkyl, which can be substituted by a hydroxyl, a di-$C_1$–$C_3$-alkylamino or an alkali metal sulfonato group, or denotes 1-isopropyl-4-methyl-cyclohex-2-yl,
X″ denotes chlorine,
m″ denotes the number 1, if $R^{1''}$ denotes hydrogen, or the number 0, if $R^{1''}$ denotes phenyl or $C_1$–$C_4$-alkyl, and
n denotes the number 0 or 1,
and, if a radical $R^{4''}$ contains an amino radical, hydrochlorides thereof.

4. Medicaments containing diethoxybis-(1-phenyl-1,3-butanedionato)-titanium(IV).

5. Compounds of the general formula $I^a$

$$[R^{1a}(CH_2)_{m^a}C(O)CR^{3a}C(O)R^{2a}]_2M^a(OR^{4a})_{2-n^a}X^a_{n^a} \quad (I^a)$$

wherein
$M^a$ denotes a number selected from the group consisting of titanium, zirconium and hafnium,
$R^{1a}$ denotes hydrogen, $C_1$–$C_8$-alkyl, or phenyl, which can be monosubstituted or polysubstituted by fluorine, chlorine, bromine, nitro, $C_1$–$C_4$-alkyl, $C_1$–$C_4$-alkoxy or trifluoromethyl,
$R^{2a}$ denotes $C_1$–$C_8$-alkyl, or phenyl, which can be monosubstituted or polysubstituted by fluorine, chlorine, bromine, nitro, $C_1$–$C_4$-alkyl, $C_1$–$C_4$-alkoxy or trifluoromethyl,
$R^{3a}$ denotes hydrogen or phenyl,
$R^{4a}$ denotes $C_1$–$C_{18}$-alkyl, which can be substituted by hydroxyl, $C_1$–$C_3$-alkylamino or alkali metal sulfonato groups, or denotes $C_5$–$C_8$-cycloalkyl, which can be substituted by $C_1$–$C_5$-alkyl groups, hydroxy or alkali metal sulfonato groups, but does not denote unsubstituted $C_1$–$C_5$- or $C_{18}$-alkyl, if $M^a$ denotes titanium, $R^{3a}$ denotes hydrogen, $m^a$ and $n^a$ denote the number 0 and $R^{1a}$ denotes $C_1$- or $C_4$-alkyl or phenyl and $R^{2a}$ denotes $C_1$–$C_6$-alkyl or phenyl, and does not denote unsubstituted

$C_3$-alkyl, if $M^a$ denotes zirconium, $R^{3a}$ denotes hydrogen, $m^a$ and $n^a$ denote the number 0 and $R^{1a}$ and $R^{2a}$ undependently from each other denote methyl or phenyl,
$X^a$ denotes fluorine, chlorine or bromine,
$m^a$ denotes the number 0 or 1, but does not denote 0 if $R^{1a}$ denotes hydrogen, and
$n^a$ denotes the number 0 or 1,
and, if $R^{4a}$ contains an amino group, hydrohalides thereof.

6. Compounds of the general formula $I^{a'}$

$$[R^{1a'}(CH_2)_{m^{a'}}C(O)CR^{3a'}C(O)R^{2a'}]_2M^{a'}(OR^{4a'})_{2-n^{a'}}X^{a'}_{n^{a'}} \quad (I^{a'})$$

wherein
$M^{a'}$ denotes titanium, zirconium or hafnium,
$R^{1a'}$ denotes hydrogen, $C_1$–$C_4$-alkyl or phenyl, which can be substituted by $C_1$–$C_2$-alkoxy,
$R^{2a'}$ denotes methyl or phenyl,
$R^{3a'}$ denotes hydrogen or phenyl,
$R^{4a'}$ denotes $C_2$–$C_{16}$-alkyl, which can be substituted by hydroxyl, di-$C_1$–$C_3$-alkylamino or an alkali metal sulfonato group, or denotes 1-isopropyl-4-methyl-cyclohex-2-yl, but does not denote unsubstituted $C_2$–$C_5$-alkyl, if $M^{a'}$ denotes titanium, $R^{3a'}$ denotes hydrogen,
$m^{a'}$ and $n^{a'}$ denote the number 0 and $R^{1a'}$ denotes $C_1$– or $C_4$-alkyl or phenyl, and does not denote unsubstituted $C_3$-alkyl, if $M^{a'}$ denotes zirconium, $R^{3a'}$ denotes hydrogen, $m^{a'}$ and $n^{a'}$ denote the number 0 and $R^{1a'}$ denotes methyl or phenyl,
$X^{a'}$ denotes chlorine,
$m^{a'}$ denotes the number 0 or 1, but not the number 0, if $R^{1a'}$ denotes hydrogen, and
$n^{a'}$ denotes the number 0 or 1,
and, if $R^{4a'}$ contains an amino group, hydrochlorides thereof.

7. Chloro-(2,3-dimethyl-2,3-butanediolato)-bis-(1-phenyl-1,3-butanedionato)-titanium(IV).
8. Chloro-(2-hydroxypropanolato)-bis-(1-phenyl-1,3-butanedionato)-titanium(IV).
9. Diethoxy-bis-(3-phenyl-2,4-pentanedionato)-titanium(IV).
10. Bis-(tridecanolato)-bis-(1-phenyl-1,3-butanedionato)-titanium(IV).
11. Process for the preparation of compounds according to claim 5 or 6, which is characterized in that

a) a tetraalkoxymetal(IV) compound $M^a(OR^{4a})_4$ or $m^{a'}(OR^{4a'})_4$, wherein $M^a$ and $M^{a'}$ and $R^{4a}$ and $R^{4a'}$ have the abovementioned meanings, is reacted with a diketone
$R^{1a}(CH_2)_{m^a}C(O)CHR^{3a}C(O)R^{2a}$ or
$R^{1a'}(CH_2)_{m^{a'}}C(O)CHR^{3a'}C(O)R^{2a'}$,
wherein $R^{1a}$ and $R^{1a'}$, $R^{2a}$ and $R^{2a'}$, $R^{3a}$ and $R^{3a'}$ and $m^a$ and $m^{a'}$ have the abovementioned meanings, in an inert solvent with exclusion of moisture, or
b) a dihalogenobis-(diketonato)-metal(IV) compound
$[R^{1a}(CH_2)_{m^a}C(O)CR^{3a}C(O)R^{2a}]_2M^aX^a_2$ or
$[R^{1a'}(CH_2)_{m^{a'}}C(O)CR^{3a'}C(O)R^{2a'}]_2M^{a'}X^{a'}_2$,
wherein $M^a$ and $M^{a'}$, $R^{1a}$ and $R^{1a'}$, $R^{2a}$ and $R^{2a'}$, $R^{3a}$ and $R^{3a'}$, $X^a$ and $X^{a'}$ and $m^a$ and $m^{a'}$ have the abovementioned meanings, is reacted with an alcohol $HOR^{4a}$ or $HOR^{4a'}$ or the corresponding alkali metal alcoholate, wherein $R^{4a}$ and $R^{4a'}$ have the abovementioned meanings, or
c) a compound of the general formula $I^a$ or $I^{a'}$, wherein $n^a$ and $n^{a'}$ denote 0 and $M^a$ and $M^{a'}$, $R^{1a}$ and $R^{1a'}$, $R^{2a}$ and $R^{2a'}$, $R^{3a}$, $R^{3a'}$, $R^{4a}$ and $R^{4a'}$ and $m^a$ and $m^{a'}$ have the abovementioned meanings, is reacted with an alcohol $HOR^{4a}$ or $HOR^{4a'}$, wherein $R^{4a}$ and has the abovementioned meanings.

12. Medicaments containing one or more complex compounds according to one or more of claims 7 to 10.
13. Coprecipitates obtained from the metal complexes of the general formula I

$$[R^1(CH_2)_mC(O)CR^3C(O)R^2]_2M(OR^4)_{2-n}X_n \quad (I)$$

wherein
M denotes titanium, zirconium or hafnium,
$R^1$ denotes hydrogen, $C_1$–$C_8$-alkyl or phenyl, which can be monosubstituted or polysubstituted by fluorine, chlorine, bromine, nitro, $C_1$–$C_4$-alkyl, $C_1$–$C_4$-alkoxy or trifluoromethyl,
$R^2$ denotes $C_1$–$C_8$-alkyl, or phenyl, which can be monosubstituted or polysubstituted by fluorine, chlorine, bromine, nitro, $C_1$–$C_4$-alkyl, $C_1$–$C_4$-alkoxy or trifluoromethyl,
$R^3$ denotes hydrogen or phenyl,
$R^4$ denotes $C_1$–$C_{18}$-alkyl, which can be substituted by hydroxyl, $C_1$–$C_3$-alkylamino or alkali metal sulfonato groups, or denotes $C_5$–$C_8$-cycloalkyl, which can be substituted by $C_1$–$C_5$-alkyl groups, hydroxyl or alkali metal sulfonato groups,
X denotes fluorine, chlorine or bromine,
m denotes the number 0 or 1, but does not denote 0 if $R^1$ denotes hydrogen, and
n denotes the number 0 or 1,
or, if a radical $R^4$ contains an amino group, hydrohalides thereof, and hydrophilic polymers.

14. Coprecipitates according to claim 13, characterized in that the hydrophilic polymer is polyvinylpyrrolidone.
15. Coprecipitates according to claim 13, characterized in that the hydrophilic polymer is a polyoxyethylene sorbitan fatty acid monoester (Tween®).
16. Coprecipitates according to claim 13, characterized in that the hydrophilic polymer is glycerol-polyethylene glycol ricinoleate.
17. Coprecipitate of diethoxybis-(1-phenyl-1,3-butanedionato)-titanium(IV) and glycerol-polyethylene glycol ricinoleate.
18. Process for the preparation of coprecipitates according to one of claims 13 to 17, characterized in that a solution of a metal complex of the general formula I

$$[R^1(CH_2)_mC(O)CR^3C(O)R^2]_2M(OR^4)_{2-n}X_n \quad (I)$$

wherein

M denotes titanium, zirconium or hafnium,
$R^1$ denotes hydrogen, $C_1$–$C_8$-alkyl or phenyl, which can be monosubstituted or polysubstituted by fluorine, chlorine, bromine, nitro, $C_1$–$C_4$-alkyl, $C_1$–$C_4$-alkoxy or trifluoromethyl,
$R^2$ denotes $C_1$–$C_8$-alkyl, or phenyl, which can be monosubstituted or polysubstituted by fluorine, chlorine, bromine, nitro, $C_1$–$C_4$-alkyl, $C_1$–$C_4$-alkoxy or trifluoromethyl,
$R^3$ denotes hydrogen or phenyl,
$R^4$ denotes $C_1$–$C_{18}$-alkyl, which can be substituted by hydroxyl, di-$C_1$–$C_3$-alkylamino or alkali metal sulfonato groups, or denotes $C_5$–$C_8$-cycloalkyl, which can be substituted by $C_1$–$C_5$-alkyl groups, hydroxyl or alkali metal sulfonato groups,
X denotes fluorine, chlorine or bromine,
m denotes the number 0 or 1, but does not denote 0 if $R^1$ denotes hydrogen, and
n denotes the number 0 or 1,
or, if a radical $R^4$ contains an amino group, hydrohalides thereof, and a hydrophilic polymer in an inert organic solvent is concentrated to dryness.

19. Process according to claim 18, characterized in that propylene glycol or butylene glycol is added to the solution of the metal complex and the hydrophilic polymer before concentration.

20. Sterile aqueous solutions containing coprecipitates according to one of claims 13 to 17.

21. Use of the metal complexes described in claims 1 to 10 for the preparation of medicaments for the treatment of cancers.

22. Use of the coprecipitates described in claims 13 to 17 for the preparation of medicaments for the treatment of cancers.

**Claims for the contracting state AT**

1. Process for the preparation of medicaments for the treatment of cancers, characterized in that one prepares metal complexes of the general formula I

$$[R^1(CH_2)_mC(O)CR^3C(O)R^2]_2M(OR^4)_{2-n}X_n \qquad (I)$$

wherein
M denotes titanium, zirconium or hafnium,
$R^1$ denotes hydrogen, $C_1$–$C_8$-alkyl or phenyl, which can be monosubstituted or polysubstituted by fluorine, chlorine, bromine, nitro, $C_1$–$C_4$-alkyl, $C_1$–$C_4$-alkoxy or trifluoromethyl,
$R^2$ denotes $C_1$–$C_8$-alkyl, or phenyl, which can be monosubstituted or polysubstituted by fluorine, chlorine, bromine, nitro, $C_1$–$C_4$-alkyl, $C_1$–$C_4$-alkoxy or trifluoromethyl,
$R^3$ denotes hydrogen or phenyl,
$R^4$ denotes $C_1$–$C_{18}$-alkyl, which can be substituted by hydroxyl, $C_1$–$C_3$-alkylamino or alkali metal sulfonato groups, or denotes $C_5$–$C_8$-cycloalkyl, which can be substituted by $C_1$–$C_5$-alkyl groups, hydroxyl or alkali metal sulfonato groups,
X denotes fluorine, chlorine or bromine,
m denotes the number 0 or 1, but does not denote 0 if $R^1$ denotes hydrogen, and
n denotes the number 0 or 1,
and, if a radical $R^4$ contains an amino group, hydrohalides thereof, by reacting

a) a tetraalkoxymetal(IV) compound $M(OR^4)_4$, wherein M and $R^4$ have the abovementioned meanings, in an inert solvent with exclusion of moisture with a diketone
$R^1(CH_2)_mC(O)CHR^3C(O)R^2$,
wherein $R^1$, $R^2$, $R^3$ and m have the abovementioned meanings, or
b) a dihalogenobis-(diketonato)-metal(IV) compound
$[R^1(CH_2)_mC(O)CR^3C(O)R^2]_2MX_2$,
wherein M, $R^1$, $R^2$, $R^3$, X and m have the abovementioned meanings, with an alcohol $HOR^4$ or the corresponding alkali metal alcoholate, wherein $R^4$ has the abovementioned meanings, or
c) a compound of the general formula I, wherein n denotes the number 0 and M, $R^1$, $R^2$, $R^3$, $R^4$ and m have the abovementioned meanings, with an alcohol $HOR^4$, wherein $R^4$ has the abovementioned meanings,
and that one or more of the resulting metal complexes of formula I are brought into a form convenient for the application as a medicament for the treatment of cancers by mixing them with a suitable pharmaceutically acceptable carrier.

2. Process for the preparation of medicaments for the treatment of cancers, characterized in that one prepares metal complexes of the general formula I'

$$[R^{1'}(CH_2)_{m'}C(O)CR^{3'}C(O)R^{2'}]_2M'(OR^{4'})_{2-n}X'_n \qquad (I)$$

wherein
M' denotes titanium, zirconium or hafnium,
$R^{1'}$ denotes hydrogen, $C_1$–$C_6$-alkyl or phenyl, which can be monosubstituted or polysubstituted by fluorine, chlorine, bromine, nitro, $C_1$–$C_2$-alkyl, $C_1$–$C_2$-alkoxy or trifluoromethyl,
$R^{2'}$ denotes $C_1$–$C_6$-alkyl or phenyl, which can be substituted by $C_1$–$C_2$-alkoxy,
$R^{3'}$ denotes hydrogen or phenyl,
$R^{4'}$ denotes $C_2$–$C_{16}$-alkyl, which can be substituted by hydroxyl, $C_1$–$C_3$-alkylamino or alkali metal sulfonato groups, or denotes cyclohexyl, which can be substituted by $C_1$–$C_4$-alkyl groups,
X' denotes fluorine or chlorine,
m' denotes the number 0 or 1, but does not denote 0 if $R^{1'}$ denotes hydrogen, and
n denotes the number 0 or 1,
and, if a radical $R^{4'}$ contains an amino group, hydrohalides thereof, by reacting

a) a tetraalkoxymetal(IV) compound $M'(OR^{4'})_4$, wherein M' and $R^{4'}$ have the abovementioned meanings, in an inert solvent with exclusion of moisture with a diketone
$R^{1'}(CH_2)_{m'}C(O)CHR^{3'}C(O)R^{2'}$,
wherein $R^{1'}$, $R^{2'}$, $R^{3'}$ and m' have the abovementioned meanings, or
b) a dihalogenobis-(diketonato)-metal(IV) compound
$[R^{1'}(CH_2)_{m'}C(O)CR^{3'}C(O)R^{2'}]_2M'X'_2$,

wherein $M'$, $R^{1'}$, $R^{2'}$, $R^{3'}$, $X'$ and $m'$ have the abovementioned meanings, with an alcohol $HOR^{4'}$ or the corresponding alkali metal alcoholate, wherein $R^{4'}$ has the abovementioned meanings, or

c) a compound of the general formula I′, wherein $n'$ denotes the number 0 and $M'$, $R^{1'}$, $R^{2'}$, $R^{3'}$, $R^{4'}$ and $m'$ have the abovementioned meanings, with an alcohol $HOR^{4'}$, wherein $R^{4'}$ has the abovementioned meanings,

and that one or more of the resulting metal complexes of formula I′ are brought into a form convenient for the application as a medicament for the treatment of cancers by mixing them with a suitable pharmaceutically acceptable carrier.

3. Process for the preparation of medicaments for the treatment of cancers, characterized in that one prepares metal complexes of the general formula I″

$$[R^{1''}(CH_2)_{m''}C(O)CR^{3''}C(O)R^{2''}]_2M''(OR^{4''})_{2-n}X''_n \quad (I'')$$

wherein

$M''$ denotes titanium, zirconium or hafnium,

$R^{1''}$ denotes hydrogen, $C_1$–$C_4$-alkyl or phenyl, which can be substituted by $C_1$–$C_2$-alkoxy,

$R^{2''}$ denotes $C_1$–$C_4$-alkyl or phenyl,

$R^{3''}$ denotes hydrogen or phenyl,

$R^{4''}$ denotes $C_2$–$C_{13}$-alkyl, which can be substituted by a hydroxyl, a di-$C_1$–$C_3$-alkylamino or an alkali metal sulfonato group, or denotes 1-isopropyl-4-methyl-cyclohex-2-yl,

$X''$ denotes chlorine,

$m''$ denotes the number 0 or 1, but does not denote 0, if $R^{1''}$ denotes hydrogen, and

n denotes the number 0 or 1,

and, if a radical $R^{4''}$ contains an amino radical, hydrochlorides thereof, by reacting

a) a tetraalkoxymetal(IV) compound $M''(OR^{4''})_4$, wherein $M''$ and $R^{4''}$ have the abovementioned meanings, in an inert solvent with exclusion of moisture with a diketone $R^{1''}(CH_2)_{m''}C(O)CHR^{3''}C(O)R^{2''}$, wherein $R^{1''}$, $R^{2''}$, $R^{3''}$ and $m''$ have the abovementioned meanings, or

b) a dihalogenobis-(diketonato)-metall(IV) compound $[R^{1''}(CH_2)_{m''}C(O)CR^{3''}C(O)R^{2''}]_2M''X''_2$, wherein $M''$, $R^{1''}$, $R^{2''}$, $R^{3''}$, $X''$ and $m''$ have the abovementioned meanings, with a alcohol $HOR^{4''}$ or the corresponding alkali metal alcoholate, wherein $R^{4''}$ has the abovementioned meanings, or

c) a compound of the general formula I″, wherein $n''$ denotes the number 0 and $M''$, $R^{1''}$, $R^{2''}$, $R^{3''}$, $R^{4''}$ and $m''$ have the abovementioned meanings, with an alcohol $HOR^{4''}$, wherein $R^{4''}$ has the abovementioned meanings,

and that one or more of the resulting metal complexes of formula I″ are brought into a form convenient for the application as a medicament for the treatment of cancers by mixing them with a suitable pharmaceutically acceptable carrier.

4. Process for the preparation of an antineoplastically effective remedy, characterized in that the complex compound diethoxybis-(1-phenyl-1,3-butanedionato)-titanium(IV) is prepared by reacting titanium(IV)tetraethoxide in an inert solvent with exclusion of moisture with benzoylacetone in a molar ratio of 1:2 and brought into a form convenient for the application as a medicament for the treatment of cancers by mixing it with a suitable pharmaceutically acceptable carrier.

5. Process for the preparation of compounds of the general formula $I^a$

$$[R^{1a}(CH_2)_{m^a}C(O)CR^{3a}C(O)R^{2a}]_2M^a(OR^{4a})_{2-n^a}X^a_{n^a} \quad (I^a)$$

wherein

$M^a$ denotes a number selected from the group consisting of titanium, zirconium and hafnium,

$R^{1a}$ denotes hydrogen, $C_1$–$C_8$-alkyl, or phenyl, which can be monosubstituted or polysubstituted by fluorine, chlorine, bromine, nitro, $C_1$–$C_4$-alkyl, $C_1$–$C_4$-alkoxy or trifluoromethyl,

$R^{2a}$ denotes $C_1$–$C_8$-alkyl, or phenyl, which can be monosubstituted or polysubstituted by fluorine, chlorine, bromine, nitro, $C_1$–$C_4$-alkyl, $C_1$–$C_4$-alkoxy or trifluoromethyl,

$R^{3a}$ denotes hydrogen or phenyl,

$R^{4a}$ denotes $C_1$–$C_{18}$-alkyl, which can be substituted by hydroxyl, $C_1$–$C_3$-alkylamino or alkali metal sulfonato groups, or denotes $C_5$–$C_8$-cycloalkyl, which can be substituted by $C_1$–$C_5$-alkyl groups, hydroxy or alkali metal sulfonato groups, but does not denote unsubstituted $C_1$–$C_5$- or $C_{18}$-alkyl, if $M^a$ denotes titanium, $R^{3a}$ denotes hydrogen, $m^a$ and $n^a$ denote the number 0 and $R^{1a}$ denotes $C_1$- or $C_4$-alkyl or phenyl and $R^{2a}$ denotes $C_1$–$C_6$-alkyl or phenyl, and does not denote unsubstituted $C_3$-alkyl, if $M^a$ denotes zirconium, $R^{3a}$ denotes hydrogen, $m^a$ and $n^a$ denote the number 0 and $R^{1a}$ and $R^{2a}$ undependently from each other denote methyl or phenyl,

$X^a$ denotes fluorine, chlorine or bromine,

$m^a$ denotes the number 0 or 1, but does not denote 0 if $R^{1a}$ denotes hydrogen, and

$n^a$ denotes the number 0 or 1,

and, if $R^{4a}$ contains an amino group, hydrohalides thereof,

which is characterized in that

a) a tetraalkoxymetal(IV) compound $M^a(OR^{4a})_4$, wherein $M^a$ and $R^{4a}$ have the abovementioned meanings, is reacted with a diketone $R^{1a}(CH_2)_{m^a}C(O)CHR^{3a}C(O)R^{2a}$, wherein $R^{1a}$, $R^{2a}$, $R^{3a}$ and $m^a$ have the abovementioned meanings, in an inert solvent with exclusion of moisture, or

b) a dihalogenobis-(diketonato)-metal(IV) compound $[R^{1a}(CH_2)_{m^a}C(O)CR^{3a}C(O)R^{2a}]_2M^aX^a_2$, wherein $M^a$, $R^{1a}$, $R^{2a}$, $R^{3a}$, $X^a$ and $m^a$ have the abovementioned meanings, is reacted with an alcohol $HOR^{4a}$ or the corresponding alkali metal alcoholate, wherein $R^{4a}$ has the abovementioned

meanings, or

c) a compound of the general formula $I^a$, wherein $n^a$ denotes the number 0 and $M^a$, $R^{1a}$, $R^{2a}$, $R^{3a}$, $R^{4a}$ and $m^a$ have the abovementioned meanings, is reacted with an alcohol $HOR^{4a}$, wherein $R^{4a}$ has the abovementioned meanings.

6. Process for the preparation of compounds of the general formula $I^{a'}$

$$[R^{1a'}(CH_2)_{m^{a'}}C(O)CR^{3a'}C(O)R^{2a'}]_2M^{a'}(OR^{4a'})_{2-n^{a'}}X^{a'}_{n^{a'}} \quad (I^{a'})$$

wherein

$M^{a'}$ denotes titanium, zirconium or hafnium,
$R^{1a'}$ denotes hydrogen, $C_1$–$C_4$-alkyl or phenyl, which can be substituted by $C_1$–$C_2$-alkoxy,
$R^{2a'}$ denotes methyl or phenyl,
$R^{3a'}$ denotes hydrogen or phenyl,
$R^{4a'}$ denotes $C_2$–$C_{16}$-alkyl, which can be substituted by hydroxyl, di-$C_1$–$C_3$-alkylamino or an alkali metal sulfonato group, or denotes 1-iso-propyl-4-methyl-cyclohex-2-yl, but does not denote unsubstituted $C_2$–$C_5$-alkyl, if $M^{a'}$ denotes titanium, $R^{3a'}$ denotes hydrogen, $m^{a'}$ and $n^{a'}$ denote the number 0 and $R^{1a'}$ denotes $C_1$- or $C_4$-alkyl or phenyl, and does not denote unsubstituted $C_3$-alkyl, if $M^{a'}$ denotes zirconium, $R^{3a'}$ denotes hydrogen, $m^{a'}$ and $n^{a'}$ denote the number 0 and $R^{1a'}$ denotes methyl or phenyl,
$X^{a'}$ denotes chlorine,
$m^{a'}$ denotes the number 0 or 1, but not the number 0, if $R^{1a'}$ denotes hydrogen, and
$n^{a'}$ denotes the number 0 or 1,
and, if $R^{4a'}$ contains an amino group, hydrochlorides thereof,

which is characterized in that

a) a tetraalkoxymetal(IV) compound $M^{a'}(OR^{4a'})_4$, wherein $M^{a'}$ and $R^{4a'}$ have the abovementioned meanings, is reacted with a diketone $R^{1a'}(CH_2)_{m^{a'}}C(O)CHR^{3a'}C(O)R^{2a'}$, wherein $R^{1a'}$, $R^{2a'}$, $R^{3a'}$ and $m^{a'}$ have the abovementioned meanings, in an invert solvent with exclusion of moisture, or

b) a dihalogenobis-(diketonato)-metal(IV) compound $[R^{1a'}(CH_2)_{m^{a'}}C(O)CR^{3a'}C(O)R^{2a'}]_2M^{a'}X^{a'}_2$, wherein $M^{a'}$, $R^{1a'}$, $R^{2a'}$, $R^{3a'}$, $X^{a'}$ and $m^{a'}$ have the abovementioned meanings, is reacted with an alcohol $HOR^{4a'}$, or the corresponding alkali metal alcoholate, wherein $R^{4a'}$ has the abovementioned meanings, or

c) a compound of the general formula $I^{a'}$, wherein $n^{a'}$ denotes the number 0 and $M^{a'}$, $R^{1a'}$, $R^{2a'}$, $R^{3a'}$, $R^{4a'}$ and $m^{a'}$ have the abovementioned meanings, is reacted with an alcohol $HOR^{4a'}$, wherein $R^{4a'}$ has the abovementioned meanings.

7. Process for the preparation of coprecipitates of the metal complexes of the general formula I

$$[R^1(CH_2)_mC(O)CR^3C(O)R^2]_2M(OR^4)_{2-n}X_n \quad (I)$$

wherein

M denotes titanium, zirconium or hafnium,
$R^1$ denotes hydrogen, $C_1$–$C_8$-alkyl or phenyl, which can be monosubstituted or polysubstituted by fluorine, chlorine, bromine, nitro, $C_1$–$C_4$-alkyl, $C_1$–$C_4$-alkoxy or trifluoromethyl,
$R^2$ denotes $C_1$–$C_8$-alkyl, or phenyl, which can be monosubstituted or polysubstituted by fluorine, chlorine, bromine, nitro, $C_1$–$C_4$-alkyl, $C_1$–$C_4$-alkoxy or trifluoromethyl,
$R^3$ denotes hydrogen or phenyl,
$R^4$ denotes $C_1$–$C_{18}$-alkyl, which can be substituted by hydroxyl, $C_1$–$C_3$-alkylamino or alkali metal sulfonato groups, or denotes $C_5$–$C_8$-cycloalkyl, which can be substituted by $C_1$–$C_5$-alkyl groups, hydroxyl or alkali metal sulfonato groups,
X denotes fluorine, chlorine or bromine,
m denotes the number 0 or 1, but does not denote 0 if $R^1$ denotes hydrogen, and
n denotes the number 0 or 1,
or, if a radical $R^4$ contains an amino group, hydrohalides thereof, and hydrophilic polymers, characterized in that a solution of the metal complex and a hydrophilic polymer in an inert organic solvent is concentrated to dryness.

8. Process according to claim 7, characterized in that polyvinylpyrrolidone is used as hydrophilic polymer.

9. Process according to claim 7, characterized in that polyoxyethylene sorbitan fatty acid monoester is used as hydrophilic polymer.

10. Process according to claim 7, characterized in that glycerol-polyethylene glycol ricinoleate is used as hydrophilic polymer.

11. Process according to one of claims 7 to 10, characterized in that propylene glycol or butylene glycol are added to the solution of the metal complex and the hydrophilic polymer before the solution is concentrated.

12. Process according to one of claims 7 to 11, characterized in that as metal complex diethoxybis-(1-phenyl-1,3-butanedionato)-titanium(IV) is used.

13. Process according to one of claims 7 to 12, characterized in that ethanol is used as inert solvent.

14. Use of the metal complexes of general formulae I, I′, I″, $I^a$ and $I^{a'}$ of the claims 1, 2, 3, 5 and 6, respectively, for the preparation of medicaments for the treatment of cancers.

15. Use of diethoxybis-(1-phenyl-1,3-butanedionato)-titanium(IV) for the preparation of medicaments for the treatment of cancers.

16. Use of coprecipitates of the metal complexes of the general formula I in claim 7 with hydrophilic polymers for the preparation of medicaments for the treatment of cancers.

**Revendications pour les Etats contractement BE, CH, DE, FR, GB, IT, LI, LU, NL, SE**

1. Médicament contenant un ou plusieurs complexes de métaux de formule générale I,

$[R^1(CH_2)_mC(O)CR^3C(O)R^2]_2M(OR^4)_{2-n}X_n$ (I),

dans laquelle les symboles ont la signification respective suivante
M titane, zirconium ou hafnium,
$R^1$ hydrogène, $C_1$–$C_8$-alkyle ou phényle, qui peut être mono- ou polysubstitué par fluor, chlore, brome, nitro, $C_1$–$C_4$-alkyle, $C_1$–$C_4$-alkoxy ou trifluorométhyle,
$R^2$ $C_1$–$C_8$-alkyle ou phényle, qui peut être mono- ou polysubstitué par fluor, chlore, brome, nitro, $C_1$–$C_4$-alkyle, $C_1$–$C_4$-alkoxy ou trifluorométhyle,
$R^3$ hydrogène ou phényle,
$R^4$ $C_1$–$C_{18}$-alkyle, qui peut être substitué par des groupes hydroxy, $C_1$–$C_3$-alkylamine ou alcalisulfonato, ou $C_5$–$C_8$-cycloalkyle, qui peut être substitué par des groupes $C_1$–$C_5$-alkyle, hydroxy ou des groupes alcalisulfonato,
X fluor, chlore ou brome,
m le nombre 0 ou 1, mais non 0, lorsque $R^1$ désigne l'hydrogène, et
n le nombre 0 ou 1,
et dans la mesure où un reste $R^4$ contient un groupe amino, leurs halogénohydrates.

2. Médicament contenant un ou plusieurs complexes de métaux de formule générale I',

$[R^{1'}(CH_2)_{m'}C(O)CR^{3'}C(O)R^{2'}]_2M'(OR^{4'})_{2-n}X'_n$ (I'),

dans laquelle les symboles ont la signification respective suivante
M' titane, zirconium ou hafnium,
$R^{1'}$ hydrogène, $C_1$–$C_6$-alkyle ou phényle, qui peut être mono- ou polysubstitué par fluor, chlore, brome, nitro, $C_1$–$C_2$-alkyle, $C_1$–$C_2$-alkoxy ou trifluorométhyle,
$R^{2'}$ $C_1$–$C_6$-alkyle ou phényle, qui peut être substitué par $C_1$–$C_2$-alkoxy,
$R^{3'}$ hydrogène ou phényle,
$R^{4'}$ $C_2$–$C_{16}$-alkyle, qui peut être substitué par des groupes hydroxy, $C_1$–$C_3$-alkylamine ou alcalisulfonato, ou cyclohexyle, qui peut être substitué par des groupes $C_1$–$C_4$-alkyle,
X' fluor, ou chlore,
m' le nombre 0 ou 1, mais non 0, lorsque le $R^{1'}$ désigne l'hydrogène, et
n le nombre 0 ou 1,
et dans la mesure où un reste $R^{4'}$ contient un groupe amino, leurs halogénohydrates.

3. Médicament contenant un ou plusieurs complexes de métaux de formule générale I'',

$[R^{1''}(CH_2)_{m''}C(O)CR^{3''}C(O)R^{2''}]_2M''(OR^{4''})_{2-n}X''_n$ (I''),

dans laquelle les symboles ont la signification respective suivante
M'' titane, zirconium ou hafnium,
$R^{1''}$ hydrogène, $C_1$–$C_4$-alkyle ou phényle, qui peut être substitué par $C_1$–$C_2$-alkoxy,
$R^{2''}$ $C_1$–$C_4$-alkyle ou phényle,
$R^{3''}$ hydrogène ou phényle,
$R^{4''}$ $C_2$–$C_{13}$-alkyle, qui peut être substitué par un hydroxy, une di-$C_1$–$C_3$-alkylamine ou un groupe alcalisulfonato, ou 1-isopropyl-4-méthyl-cyclohexyle-2,
X'' chlore,
m'' le nombre 1, lorsque $R^{1''}$ désigne l'hydrogène, et le nombre 0, lorsque $R^{1''}$ désigne phényle ou $C_1$–$C_4$-alkyle,
n le nombre 0 ou 1 et, dans la mesure où un reste $R^{4''}$
contient un groupe amino, leurs chlorhydrates.

4. Médicament contenant le diéthoxy bis-(1-phényl-1,3-butanedionato)-titane(IV).

5. Composés de formule générale $I^a$,

$[R^{1a}(CH_2)_{m^a}C(O)CR^{3a}C(O)R^{2a}]_2M^a(OR^{4a})_{2-n^a}X^a_{n^a}$ ($I^a$),

dans laquelle les symboles ont la signification respective suivante
$M^a$ titane, zirconium ou hafnium
$R^{1a}$ hydrogène, $C_1$–$C_8$-alkyle ou phényle, qui peut être mono- ou polysubstitué par fluor, chlore, brome, nitro, $C_1$–$C_4$-alkyle, $C_1$–$C_4$-alkoxy ou trifluorométhyle,
$R^{2a}$ $C_1$–$C_8$ alkyle ou phényle, qui peut être mono- ou polysubstitué par fluor, chlore, brome, nitro, $C_1$–$C_4$-alkyle, $C_1$–$C_4$-alkoxy ou trifluorométhyle,
$R^{3a}$ hydrogène ou phényle,
$R^{4a}$ $C_1$–$C_{18}$-alkyle, qui peut être substitué par des groupes hydroxy, $C_1$–$C_3$-alkylamine ou alcalisulfonato, ou $C_5$–$C_8$-cycloalkyle, qui peut être substitué par des groupes $C_1$–$C_5$-alkyle, hydroxy ou des groupes alcalisulfonato, toutefois, $C_1$–$C_5$- ou $C_{18}$-alkyle ne peuvent pas être non substitués lorsque les symboles ont la signification respective suivante: $M^a$ titane, $R^{3a}$ hydrogène, $m^a$ et $n^a$ le nombre 0 et $R^{1a}$ $C_1$- ou $C_4$-alkyle ou phényle et $R^{2a}$ $C_1$–$C_6$-alkyle ou phényle, et $C_3$-alkyle ne peut pas être non substitué lorsque les symboles ont la signification respective suivante: $M^a$ zirconium, $R^{3a}$ hydrogène, $m^a$ et $n^a$ le nombre 0 et $R^{1a}$ et $R^{2a}$ désignent indépendamment l'un de l'autre méthyle ou phényle,
$X^a$ fluor, chlore ou brome,
$m^a$ le nombre 0 ou 1, mais non 0, lorsque le $R^{1a}$ désigne l'hydrogène, et
$n^a$ le nombre 0 ou 1,
et dans la mesure où un reste $R^{4a}$ contient un groupe amino, leurs halogénohydrates.

6. Composés de formule générale $I^{a'}$

$[R^{1a'}(CH_2)_{m^{a'}}C(O)CR^{3a'}C(O)R^{2a'}]_2M^{a'}(OR^{4a'})_{2-n^{a'}}X^{a'}_{n^{a'}}$ ($I^{a'}$),

dans laquelle les symboles ont la signification respective
$M^{a'}$ titane, zirconium ou hafnium,
$R^{1a'}$ hydrogène, $C_1$–$C_4$-alkyle ou phényle, qui peut être substitué par $C_1$–$C_2$-alkoxy,
$R^{2a'}$ méthyle ou phényle,
$R^{3a'}$ hydrogène ou phényle,
$R^{4a'}$ $C_2$–$C_{16}$-alkyle, qui peut être substitué par hydroxy, di-$C_1$–$C_3$-alkylamine ou un groupe alcalisulfonato, ou 1-isopropyl-4-méthyl-cyclohexyle-2, toutefois $C_2$–$C_5$-alkyle, ne peuvent pas être non substitués lorsque les symboles ont la signification respective suivante: $M^{a'}$ titane, $R^{3a'}$ hydrogène, $m^{a'}$ et $n^{a'}$ le nombre 0 et $R^{1a'}$, $C_1$- ou $C_4$-alkyle ou phényle, et $C_3$-alkyle ne peut pas être non substitué, lorsque les symboles ont la signification respective suivante: $M^{a'}$ zirconium, $R^{3a'}$ hydrogène, $m^{a'}$ et $n^{a'}$ le nombre 0 et $R^{1a'}$ méthyle ou phényle,
$X^{a'}$ chlore,
$m^{a'}$ le nombre 0 ou 1, toutefois pas 0 lorsque $R^{1a'}$ désigne l'hydrogène, et
$n^{a'}$ le nombre 0 ou 1,
et, dans la mesure où $R^{4a'}$ contient un groupe amino, leurs chlorhydrates.

7. Chloro-(2,3-diméthyl-2,3-butanediolato)-bis-(1-phényl-1,3-butanedionato)-titane(IV).

8. Chloro-(2-hydroxypropanolato)-bis-(1-phényl-1,3-butanedionato)-titane(IV).

9. Diéthoxy-bis-(3-phényl-2,4-pentanedionato)-titane(IV).

10. Bis-(tridécanolato)-bis-(1-phényl-1,3-butanedionato)-titane(IV).

11. Procédé pour la préparation des composés selon la revendication 5 ou 6, caractérisé en ce que

a) on fait réagir un composé tétraalkoxymétal(IV) $M^a(OR^{4a})_4$ ou $M^{a'}(OR^{4a'})_{4'}$, dans lesquelles $M^a$ ou $M^{a'}$, $R^{4a}$ ou $R^{4a'}$ sont définis comme spécifié ci-dessus, sous exclusion d'humidité dans un solvant inerte avec une dicétone
$R^{1a}(CH_2)_{m^a}C(O)CHR^{3a}C(O)R^{2a}$ ou
$R^{1a'}(CH_2)_{m^{a'}}C(O)CHR^{3a'}C(O)R^{2a'}$,
dans lesquelles $R^{1a}$ ou $R^{1a'}$, $R^{2a}$ ou $R^{2a'}$, $R^{3a}$ ou $R^{3a'}$ et $m^a$ ou $m^{a'}$ sont définis comme spécifié ci-dessus,
b) on fait réagir un composé dihalogénobis-(dicétonato)-métal(IV)
$[R^{1a}(CH_2)_{m^{a'}}C(O)CR^{3a}C(O)R^{2a}]_2M^aX^a{}_2$ ou
$[R^{1a'}(CH_2)_{m^{a'}}C(O)CR^{3a'}C(O)R^{2a'}]_2M^{a'}X^{a'}{}_2$,
dans laquelle $M^a$ ou $M^{a'}$, $R^{1a}$ ou $R^{1a'}$, $R^{2a}$ ou $R^{2a'}$, $R^{3a}$ ou $R^{3a'}$, $X^a$ ou $X^{a'}$ et $m^a$ ou $m^{a'}$ sont définis comme spécifié ci-dessus, avec un alcool $HOR^{4a}$ ou $HOR^{4a'}$ ou l'alcoolate alcalin correspondant, dans lesquelles $R^{4a}$ ou $R^{4a'}$ sont définis comme spécifié ci-dessus, ou
c) on fait réagir un composé de formule générale $I^a$ ou $I^{a'}$, dans lesquelles $n^a$ ou $n^{a'}$ désignent 0 et $M^a$ ou $M^{a'}$, $R^{1a}$ ou $R^{1a'}$, $R^{2a}$ ou $R^{2a'}$, $R^{3a}$ ou $R^{3a'}$, $R^{4a}$ ou $R^{4a'}$ et $m^a$ ou $m^{a'}$ sont définis comme spécifié ci-dessus, avec un alcool $HOR^{4a}$ ou $HOR^{4a'}$, dans lesquelles $R^{4a}$ ou $R^{4a'}$ sont définis comme spécifié ci-dessus.

12. Médicament contenant un ou plusieurs composés complexes selon une ou plusieurs des revendications 7 à 10.

13. Coprécipités obtenus à partir des complexes de métaux de formule générale I et des polymères hydrophiles.

$$[R^1(CH_2)_mC(O)CR^3C(O)R^2]_2M(OR^4)_{2-n}X_n \quad (I),$$

dans laquelle les symboles ont la signification respective suivante
M titane, zirconium ou hafnium,
$R^1$ hydrogène, $C_1$–$C_8$-alkyle ou phényle, qui peut être mono- ou polysubstitué par fluor, chlore, brome, nitro, $C_1$–$C_4$-alkyle, $C_1$–$C_4$-alkoxy ou trifluorométhyle,
$R^2$ $C_1$–$C_8$-alkyle ou phényle, qui peut être mono- ou polysubstitué par fluor, chlore, brome, nitro, $C_1$–$C_4$-alkyle, $C_1$–$C_4$-alkoxy ou trifluorométhyle,
$R^3$ hydrogène ou phényle,
$R^4$ $C_1$–$C_{18}$-alkyle, qui peut ête substitué par des groupes hydroxy, $C_1$–$C_3$-alkylamine ou alcalisulfonato, ou $C_5$–$C_8$-cycloalkyle, qui peut être substitué par des groupes $C_1$–$C_5$-alkyle, hydroxy ou des groupes alcalisulfonato,
X fluor, chlore ou brome,
m le nombre 0 ou 1, mais non 0, lorsque le $R^1$ désigne l'hydrogène, et
n le nombre 0 ou 1,
et dans la mesure où un reste $R^4$ contient un groupe amino, leurs halogénohydrates.

14. Coprécipités selon la revendication 13, caractérisé en ce qu'il s'agit, en ce que concerne le polymère hydrophile, de la polyvinylpyrrolidone.

15. Coprécipités selon la revendication 13, caractérisé en ce qu'il s'agit, en ce qui concerne le polymère hydrophile, d'un mono-ester d'acide gras de polyoxyéthylène sorbitan (mis sur le marché sous le nom de Tween).

16. Coprécipités selon la revendication 13, caractérisés en ce qu'il s'agit, en ce qui concerne le polymère hydrophile de ricinoléate de glycérine-polyéthylène glycol.

17. Coprécipité de diéthoxybis-(1-phényl-1,3-butanedionato)-titane(IV) et de ricinoléate de glycérine-polyéthylène glycol (mis sur le marché sous le nom de Crémophor EL).

18. Procédé pour la préparation des coprécipités selon l'une des revendications 13 à 17, caractérisé en ce que l'on concentre à siccité dans un solvant organique inerte une solution d'un complexe de métal de formule générale (I) et un polymère hydrophile,

$$[R^1(CH_2)_mC(O)CR^3C(O)R^2]_2M(OR^4)_{2-n}X_n \quad (I),$$

dans laquelle les symboles ont la signification respective suivante
M titane, zirconium ou hafnium,
$R^1$ hydrogène, $C_1$–$C_8$-alkyle ou phényle, qui peut être mono- ou polysubstitué par fluor, chlore, brome, nitro, $C_1$–$C_4$-alkyle, $C_1$–$C_4$-alkoxy ou trifluorométhyle,
$R^2$ $C_1$–$C_8$-alkyle ou phényle, qui peut être mono- ou polysubstitué par fluor, chlore, brome, nitro, $C_1$–$C_4$-akyle, $C_1$–$C_4$-alkoxy ou trifluorométhyle,
$R^3$ hydrogène ou phényle,
$R^4$ $C_1$–$C_{18}$-alkyl, qui peut être substitué par des groupes hydroxy, $C_1$–$C_3$-alkylamine ou alcalisulfonato, ou $C_5$–$C_8$-cycloalkyle, qui peut être substitué par des groupes $C_1$–$C_5$-alkyle, hydroxy ou des groupes alcalisulfonato,
X fluor, chlore ou brome,

m le nombre 0 ou 1, mais non 0, lorsque le $R^1$ désigne l'hydrogène, et
n le nombre 0 ou 1,
et dans la mesure où un reste $R^4$ contient un groupe amino, leurs halogénohydrates.

19. Procédé selon la revendication 18, caractérisé en ce que l'on ajoute du propylèneglycol ou du butylèneglycol à la solution du complexe de métal et du polymère hydrophile avant la concentration.

20. Solutions aqueuses stériles contenant des coprécipités selon l'une des revendications 13 à 17.

21. Application des complexes de métaux mentionnés dans les revendications 1 à 10 à la préparation de médicaments pour le traitement des maladies cancéreuses.

22. Application des coprécipités indiqués dans les revendications 13 à 17 à la préparation de médicaments pour le traitement des maladies cancéreuses.

**Revendications pour l'Etat contractant AT**

1. Procédé pour la préparation de médicaments pour le traitement des maladies cancéreuses, caractérisé en ce que l'on prépare des complexes de métaux de formule générale I

$$[R^1(CH_2)_mC(O)CR^3C(O)R^2]_2M(OR^4)_{2-n}X_n \quad (I),$$

dans laquelle les symboles ont la signification respective suivante
M titane, zirconium ou hafnium,
$R^1$ hydrogène, $C_1$–$C_8$-alkyle ou phényle, qui peut être mono- ou polysubstitué par fluor, chlore, brome, nitro, $C_1$–$C_4$-alkyle, $C_1$–$C_4$-alkoxy ou trifluorométhyle,
$R^2$ $C_1$–$C_8$-alkyle ou phényle, qui peut être mono- ou polysubstitué par fluor, chlore, brome, nitro, $C_1$–$C_4$-alkyle, $C_1$–$C_4$-alkoxy ou trifluorométhyle,
$R^3$ hydrogène ou phényle,
$R^4$ $C_1$–$C_{18}$-alkyle, qui peut être substitué par des groupes hydroxy, $C_1$–$C_3$-alkylamine ou alcalisulfonato, ou $C_5$–$C_8$-cycloalkyle, qui peut être substitué par des groupes $C_1$–$C_5$-alkyle, hydroxy ou des groupes alcalisulfonato,
X fluor, chlore ou brome,
m le nombre 0 ou 1, mais non 0, lorsque le $R^1$ désigne l'hydrogène, et
n le nombre 0 ou 1,
ou dans la mesure où un reste $R^4$ contient un groupe amino, leurs halogénohydrates, en ce que

a) on fait réagir un composé tétraalkoxymétal(IV) $M(OR^4)_4$, dans laquelle M et $R^4$ qui sont définis comme spécifié ci-dessus, sous exclusion d'humidité dans un solvant inerte avec une dicétone
$R^1(CH_2)_mC(O)CHR^3C(O)R^2$,
dans laquelle $R^1$, $R^2$, $R^3$ et m sont définis comme spécifié ci-dessus,
b) on fait réagir un composé dihalogénobis-(dicétonato)-métal(IV)
$[R^1(CH_2)_mC(O)CR^3C(O)R^2]_2MX_2$,
dans laquelle M, $R^1$, $R^2$, $R^3$, X et m sont définis comme spécifiés ci-dessus, avec un alcool $HOR^4$ ou l'alcoolate alcalin, dans laquelle $R^4$ défini comme spécifié ci-dessus, ou
c) on fait réagir un composé de formule générale I, dans laquelle n désigne le nombre 0 et M, $R^1$, $R^2$, $R^3$, $R^4$ et m sont définis comme spécifié ci-dessus, avec un alcool $HOR^4$, dans laquelle $R^4$ est défini comme spécifié ci-dessus,

et que l'on transforme un ou plusieurs des complexes de métaux de formule I ainsi obtenus par mélange avec un véhicule pharmaceutiquement acceptable approprié sous une forme convenable en vue de leur administration comme médicament pour le traitement des maladies cancéreuses.

2. Procédé pour la préparation de médicaments pour le traitement des maladies cancéreuses, caractérisé en ce qu'on prépare des complexes de métaux de formule générale I'

$$[R^{1'}(CH_2)_{m'}C(O)CR^{3'}C(O)R^{2'}]_2M'(OR^{4'})_{2-n}X'_n \quad (I')$$

dans laquelle les symboles ont la signification respective suivante
M' titane, zirconium ou hafnium,
$R^{1'}$ hydrogène, $C_1$–$C_6$-alkyle ou phényle, qui peut être mono- ou polysubstitué par fluor, chlore, brome, nitro, $C_1$–$C_2$-alkyle, $C_1$–$C_2$-alkoxy ou trifluorométhyle,
$R^{2'}$ $C_1$–$C_6$-alkyle ou phényle, qui peut être substitué par $C_1$–$C_2$-alkoxy,
$R^{3'}$ hydrogène ou phényle,
$R^{4'}$ $C_2$–$C_{16}$-alkyle, qui peut être substitué par des groupes hydroxy, $C_1$–$C_3$ alkylamine ou alcalisulfonato, ou cyclohexyle, qui peut être substitué par des groupes $C_1$–$C_4$-alkyle,
X' Fluor, chlore,
m' le nombre 0 ou 1, mais non 0, lorsque le $R^{1'}$ désigne l'hydrogène, et
n le nombre 0 ou 1,
ou dans la mesure où un reste $R^{4'}$ contient un groupe amino, leurs halogénohydrates, en ce que

a) on fait réagir un composé tétraalkoxymétal(IV) $M'(OR^{4'})_4$, dans laquelle M' et $R^{4'}$ qui sont définis comme spécifié ci-dessus, sous exclusion d'humidité dans un solvant inerte avec une dicétone
$R^{1'}(CH_2)_mC(O)CHR^{3'}C(O)R^{2'}$,
dans laquelle $R^{1'}$, $R^{2'}$, $R^{3'}$ et m' sont définis comme spécifié ci-dessus,
b) on fait réagir un composé dihalogénobis-(dicétonato)-métal(IV)
$[R^{1'}(CH_2)_{m'}C(O)CR^{3'}C(O)R^{2'}]_2M'X'_2$,
dans laquelle M', $R^{1'}$, $R^{2'}$, $R^{3'}$, X' et m' sont définis comme spécifié ci-dessus, avec un alcool $HOR^{4'}$ ou l'alcoolate alcalin correspondant, dans laquelle $R^{4'}$ est défini comme spécifié ci-dessus, ou
c) on fait réagir un composé de formule générale

I', dans laquelle n' désigne le nombre 0 et M', $R^{1'}$, $R^{2'}$, $R^{3'}$, $R^{4'}$ et m' sont définis comme spécifié ci-dessus, avec un alcool $HOR^{4'}$, dans laquelle $R^{4'}$ est défini comme spécifié ci-dessus,

et que l'on transforme un ou plusieurs des complexes de métaux de formule I' ainsi obtenus par mélange avec un véhicule pharmaceutiquement acceptable approprié sous une forme convenable en vue de leur administration comme médicament pour le traitement des maladies cancéreuses.

3. Procédé pour la préparation de médicaments pour le traitement des maladies cancéreuses, caractérisé en ce qu'on prépare des complexes de métaux de formule générale I''

$$[R^{1''}(CH_2)_{m''}C(O)CR^{3''}C(O)R^{2''}]_2M''(OR^{4''})_{2-n}X''_n \quad (I''),$$

dans laquelle les symboles ont la signification respective suivante
M'' titane, zirconium ou hafnium,
$R^{1''}$ hydrogène, $C_1$–$C_4$-alkyle ou phényle, qui peut être substitué par $C_1$–$C_2$-alkoxy,
$R^{2''}$ $C_1$–$C_4$-alkyle ou phényle,
$R^{3''}$ hydrogène ou phényle,
$R^{4''}$ $C_2$–$C_{13}$-alkyle, qui peut être substitué par un hydroxy, une di-$C_1$–$C_3$-alkylamine ou un groupe alcalisulfonato, ou 1-isopropyl-4-méthyl-cyclohexyl-2,
X'' chlore,
m'' le nombre 0 ou 1, mais non le nombre 0 lorsque $R^{1''}$ désigne hydrogène
n le nombre 0 ou 1
ou dans la mesure où $R^{4''}$ contient un groupe amino, leurs halogénohydrates, en ce que

a) on fait réagir un composé tétraalkoxymétal(IV)$M''(OR^{4''})_4$, dans laquelle M'' et $R^{4''}$ qui sont définis comme spécifié ci-dessus, sous exclusion d'humidité dans un solvant inerte avec une dicétone
$R^{1''}(CH_2)_{m''}C(O)CHR^{3''}C(O)R^{2''}$,
dans laquelle $R^{1''}$, $R^{2''}$, $R^{3''}$ et m'' sont définis comme spécifié ci-dessus,
b) on fait réagir un composé dihalogénobis-(dicétonato)-métal(IV)
$[R^{1''}(CH_2)_{m''}C(O)CR^{3''}C(O)R^{2''}]_2M''X''_2$,
dans laquelle M'', $R^{1''}$, $R^{2''}$, $R^{3''}$, X'' et m'' sont définis comme spécifié ci-dessus, avec un alcool $HOR^{4''}$ ou l'alcoolate alcalin correspondant, dans laquelle $R^{4''}$ et m'' sont définis comme spécifié ci-dessus, ou
c) on fait réagir un composé de formule générale I'', dans laquelle n désigne le nombre 0 et M, $R^1$, $R^2$, $R^3$, $R^4$ et m'' sont définis comme spécifié ci-dessus, avec un alcool $HOR^{4''}$, dans laquelle $R^{4''}$ est défini comme spécifié ci-dessus,

et que l'on transforme un ou plusieurs des complexes de métaux de formule I'' ainsi obtenus par mélange avec un véhicule pharmaceutiquement acceptable approprié sous une forme convenable en vue de leur administration comme médicament pour le traitement des maladies cancéreuses.

4. Procédé pour la préparation d'agents à activité antinéoplastique, caractérisé en ce que l'on prépare le composé complexe diéthoxybis(benzoylacétonato)titane(IV), en ce que l'on fait réagir le tétraéthoxyde de titane(IV) sous exclusion d'humidité dans un solvant inerte, avec la benzoylacétone dans le rapport molaire de 1:2, et que l'on transforme le diéthoxybis(benzoylacétonato)titane(IV) par mélange avec un véhicule pharmaceutique acceptable approprié en une forme convenable pour l'administration comme médicament pour le traitement des maladies cancéreuses.

5. Procédé pour la préparation des composés de formule $I^a$

$$[R^{1a}(CH_2)_{m^a}C(O)CR^{3a}C(O)R^{2a}]_2M^a(OR^{4a})_{2-n^a}X^a_{n^a} \quad (I^a),$$

dans laquelle les symboles ont la signification respective suivante
$M^a$ titane, zirconium ou hafnium,
$R^{1a}$ hydrogène, $C_1$–$C_8$-alkyle ou phényle, qui peut être mono- ou polysubstitué par fluor, chlore, brome, nitro, $C_1$–$C_4$-alkyle, $C_1$–$C_4$-alkoxy ou trifluorométhyle,
$R^{2a}$ $C_1$–$C_8$-alkyle ou phényle, qui peut être mono- ou polysubstitué par fluor, chlore, brome, nitro, $C_1$–$C_4$-alkyle, $C_1$–$C_4$-alkoxy ou trifluorométhyle,
$R^{3a}$ hydrogène ou phényle,
$R^{4a}$ $C_1$–$C_{18}$-alkyle, qui peut être substitué par des groupes hydroxy, $C_1$–$C_3$-alkylamine ou alcalisulfonato, ou $C_5$–$C_8$-cycloalkyle, qui peut être substitué par des groupes $C_1$–$C_5$-alkyle, hydroxy ou des groupes alcalisulfonato, toutefois, $C_1$–$C_5$- ou $C_{18}$-alkyle ne peuvent pas être non substitués lorsque les symboles ont la signification respective suivante: $M^a$ titane, $R^{3a}$ hydrogène, $m^a$ et $n^a$ le nombre 0 et $R^{1a}$ $C_1$- ou $C_4$-alkyle ou phényle et $R^{2a}$ $C_1$–$C_6$-alkyle ou phényle, et $C_3$-alkyle ne peut pas être non substitué lorsque les symboles ont la signification respective suivante: $M^a$ zirconium, $R^{3a}$ hydrogène, $m^a$ et $n^a$ le nombre 0 et $R^{1a}$ et $R^{2a}$ désignent indépendamment l'un de l'autre méthyle ou phényle,
$X^a$ fluor, chlore ou brome,
$m^a$ le nombre 0 ou 1, mais non 0, lorsque le $R^{1a}$ désigne l'hydrogène, et
$n^a$ le nombre 0 ou 1,
ou dans la mesure où un reste $R^{4a}$ contient un groupe amino, leurs halogénohydrates, caractérisé en ce que

a) on fait réagir un composé tétraalkoxymétal(IV)
$M^a(OR^{4a})_4$, dans laquelle $M^a$ et $R^{4a}$ qui sont définis comme spécifié ci-dessus, sous exclusion d'humidité dans un solvant inerte avec une dicétone
$R^{1a}(CH_2)_{m^a}C(O)CHR^{3a}C(O)R^{2a}$,
dans laquelle $R^{1a}$, $R^{2a}$, $R^{3a}$ et $m^a$ sont définis comme spécifié ci-dessus,
b) on fait réagir un composé dihalogénobis-(di-

cétonato)-métal(IV)
$[R^{1a}(CH_2)_{m^a}C(O)CR^{3a}C(O)R^{2a}]_2M^aX^a{}_2$,
dans laquelle $M^a$, $R^{1a}$, $R^{2a}$, $R^{3a}$, $X^a$ et $m^a$ sont définis comme spécifié ci-dessus avec un alcool $HOR^{4a}$ ou l'alcoolate alcalin correspondant, dans laquelle $R^{4a}$ est défini comme spécifié ci-dessus, ou

c) on fait réagir un composé de formule générale $I^a$, dans laquelle $n^a$ désigne le nombre 0 et $M^a$, $R^{1a}$, $R^{2a}$, $R^{3a}$, $R^{4a}$ et $m^a$ sont définis comme spécifié ci-dessus, avec un alcool $HOR^{4a}$, dans laquelle $R^{4a}$ est défini comme spécifié ci-dessus.

6. Procédé pour la préparation des composés de formule générale $I^{a'}$,

$$[R^{1a'}(CH_2)_{m^{a'}}C(O)CR^{3a'}C(O)R^{2a'}]_2M^{a'}(OR^{4a'})_{2-n^{a'}}X^{a'}{}_{n^{a'}} \quad (I^{a'}),$$

dans laquelle les symboles ont la signification respective

$M^{a'}$ titane, zirconium ou hafnium,
$R^{1a'}$ hydrogène, $C_1$–$C_4$-alkyle ou phényle, qui peut être substitué par $C_1$–$C_2$-alkoxy,
$R^{2a'}$ méthyle ou phényle,
$R^{3a'}$ hydrogène ou phényle,
$R^{4a'}$ $C_2$–$C_{16}$-alkyle, qui peut être substitué par hydroxy, di-$C_1$–$C_3$-alkylamine ou un groupe alcalisulfonato, ou 1-isopropyl-4-méthyl-cyclohexyle-2, toutefois $C_2$–$C_5$-alkyle ne peuvent pas être non substitués lorsque les symboles ont la signification respective suivante: $M^{a'}$ titane, $R^{3a'}$ hydrogène, $m^{a'}$ et $n^{a'}$ le nombre 0 et $R^{1a'}$ $C_1$- ou $C_4$-alkyle ou phényle, et $C_3$-alkyle ne peut pas être non substitué, lorsque les symboles ont la signification respective suivante $M^{a'}$ zirconium, $R^{3a'}$ hydrogène, $m^{a'}$ et $n^{a'}$ le nombre 0 et $R^{1a'}$ méthyle ou phényle,
$X^{a'}$ chlore,
$m^{a'}$ le nombre 0 ou 1, toutefois pas 0, lorsque $R^{1a'}$ désigne l'hydrogène, et
$n^{a'}$ le nombre 0 ou 1,
ou dans la mesure où $R^{4a'}$ contient un groupe amino, leurs chlorhydrates, caractérisé en ce que

a) on fait réagir un composé tétraalkoxymétal(IV)
$M^{a'}(OR^{4a'})_4$, dans laquelle $M^{a'}$ et $R^{4a'}$ qui sont définis comme spécifié ci-dessus. Sous exclusion d'humidité dans un solvant inerte avec une dicétone
$R^{1a'}(CH_2)_{m^{a'}}C(O)CHR^{3a'}C(O)R^{2a'}$,
dans laquelle $R^{1a'}$, $R^{2a'}$, $R^{3a'}$ et $m^{a'}$ sont définis comme spécifié ci-dessus,

b) on fait réagir un composé dihalogénobis-(dicétonato)-métal(IV)
$[R^{1a'}(CH_2)_{m^{a'}}C(O)CR^{3a'}C(O)R^{2a'}]_2M^{a'}X^{a'}{}_2$,
dans laquelle $M^{a'}$, $R^{1a'}$, $R^{2a'}$, $R^{3a'}$, $X^{a'}$ et $m^{a'}$ sont définis comme spécifié ci-dessus avec un alcool $HOR^{4a}$ ou l'alcoolate alcalin correspondant, dans laquelle $R^{4a'}$ est défini comme spécifié ci-dessus, ou

c) on fait réagir un composé de formule générale $I^{a'}$, dans laquelle $n^{a'}$ désigne le nombre 0 et $M^{a'}$, $R^{1a'}$, $R^{2a'}$, $R^{3a'}$ et $m^{a'}$ sont définis comme spécifié ci-dessus, avec un alcool $HOR^{4a'}$, dans laquelle $R^{4a'}$ est défini comme spécifié ci-dessus,

7. Procédé pour la préparation de coprécipités des complexes de métaux de formule générale I,

$$[R^1(CH_2)_mC(O)CR^3C(O)R^2]_2M(OR^4)_{2-n}X_n \quad (I),$$

dans laquelle les symboles ont la signification respective suivante
M titane, zirconium ou hafnium
$R^1$ hydrogène, $C_1$–$C_8$-alkyle ou phényle, qui peut être mono- ou polysubstitué par fluor, chlore, brome, nitro, $C_1$–$C_4$-alkyle, $C_1$–$C_4$-alkoxy ou trifluorméthyle,
$R^2$ $C_1$–$C_8$-alkyle ou phényle, qui peut être mono- ou polysubstitué par fluor, chlore, brome, nitro, $C_1$–$C_4$-alkyle, $C_1$–$C_4$-alkoxy ou trifluorométhyle,
$R^3$ hydrogène ou phényle,
$R^4$ $C_1$–$C_{18}$-alkyle, qui peut être substitué par des groupes hydroxy, $C_1$–$C_3$-alkylamine ou alcalisulfonato, ou $C_5$–$C_8$-cycloalkyle, qui peut être substitué par des groupes $C_1$–$C_5$-alkyle, hydroxy ou des groupes alcalisulfonato,
X fluor, chlore ou brome,
m le nombre 0 ou 1, mais non 0, lorsque le $R^1$ désigne l'hydrogène, et
n le nombre 0 ou 1,
ou dans la mesure où un reste $R^4$ contient un groupe amino, leurs halogénohydrates, avec des polymères hydrophiles, caractérisé en ce qu'on concentre à siccité une solution du complexe de métal et d'un polymère hydrophile dans un solvant organique inerte.

8. Procédé selon la revendication 7, caractérisé en ce qu'on utilise comme polymère hydrophile la polyvinylpyrrolidone.

9. Procédé selon la revendication 7, caractérisé en ce qu'on utilise comme polymère hydrophile un monoester d'acide gras de polyoxyéthylène sorbitan.

10. Procédé selon la revendication 7, caractérisé en ce que l'on utilise comme polymère hydrophile un ricinoléate de glycérine-polyéthylène glycol.

11. Procédé selon l'une des revendications 7 à 10, caractérisé en ce qu'on ajoute du propylèneglycol ou du butylèneglycol à la solution du complexe de métal et du polymère hydrophile avant la concentration.

12. Procédé selon l'une des revendications 7 à 11, caractérisé en ce que l'on utilise comme complexe de métal le diéthoxybis(benzoylacétonato) titane(IV).

13. Procédé selon l'une des revendications 7 à 12, caractérisé en ce qu'on utilise l'éthanol comme solvant inerte.

14. Application des complexes de métaux des formules générales I, I', I", $I^a$ et $I^{a'}$ spécifiées dans les revendications 1, 2, 3, 5 et 6 à la préparation de médicaments pour le traitement de maladies cancéreuses.

15. Application du diéthoxybis-(1-phényl-1,3-butanedionato)titane(IV) à la préparation de médicaments pour le traitement des maladies cancéreuses.

16. Applications des coprécipités des complexes de métaux de formule générale I spécifiée dans la revendication 7 avec des polymères hydrophiles à la préparation de médicaments pour le traitement des maladies cancéreuses.